(19) Europäisches Patentamt / European Patent Office / Office européen des brevets

(11) **EP 4 209 773 A1**

(12) **EUROPEAN PATENT APPLICATION**
published in accordance with Art. 153(4) EPC

(43) Date of publication:
**12.07.2023 Bulletin 2023/28**

(21) Application number: **22861655.3**

(22) Date of filing: **22.08.2022**

(51) International Patent Classification (IPC):
$G01N\ 3/00$ (2006.01)     $G01N\ 3/06$ (2006.01)
$G01N\ 3/08$ (2006.01)     $G01N\ 3/24$ (2006.01)
$G01N\ 3/60$ (2006.01)     $G01N\ 33/44$ (2006.01)

(52) Cooperative Patent Classification (CPC):
G01N 3/00; G01N 3/06; G01N 3/08; G01N 3/24;
G01N 3/60; G01N 33/44

(86) International application number:
**PCT/KR2022/012499**

(87) International publication number:
**WO 2023/027445 (02.03.2023 Gazette 2023/09)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: 27.08.2021  KR 20210113915
19.10.2021  KR 20210138918
13.07.2022  KR 20220086165

(71) Applicant: **Lg Chem, Ltd.**
**Seoul 07336 (KR)**

(72) Inventors:
• **MOON, Sung Nam**
**Daejeon 34122 (KR)**
• **KIM, Hyun Tae**
**Daejeon 34122 (KR)**
• **JEON, Sang Jin**
**Daejeon 34122 (KR)**
• **JUNG, Jin Mi**
**Daejeon 34122 (KR)**
• **CHOI, Jin Uk**
**Daejeon 34122 (KR)**
• **IM, Dam Hyeok**
**Daejeon 34122 (KR)**

(74) Representative: **Goddar, Heinz J.**
**Boehmert & Boehmert**
**Anwaltspartnerschaft mbB**
**Pettenkoferstrasse 22**
**80336 München (DE)**

(54) **SYSTEM AND METHOD FOR PREDICTING PHYSICAL PROPERTIES OF MULTILAYER MATERIAL**

(57)    A system and method for predicting the physical properties of a multilayer material are provided. The system and method can predict physical properties such as a coefficient of thermal expansion and coefficient of water expansion of the multilayer material, and a warpage of the multilayer material when developing the multilayer material.

EP 4 209 773 A1

【FIG. 5】

$\alpha_{1.2}^{k}, \beta_{1.2}^{k}, \Delta T, \Delta C$ INPUT ~S21

$e_{1.2}^{k}$ ~S22

$\Theta_{k}$

$e_{x,y,s}^{k}$ ~S23

$[Q]_{x,y}^{k}$

$Z_{k}$

$N^{HT}, M^{HT}$ ~S24

$N, M$

$/N, /M$ ~S25

COEFFICIENTS OF THERMAL EXPANSION AND WATER EXPANSION OF LAMINATE ~S26

$[a]_{x,y}, [b]_{x,y}$
$[c]_{x,y}, [d]_{x,y}$

CALCULATION OF STRAINS AND CURVATURES OF MIDDLE PLANE ~S27

SAMPLE SIZE

WARPAGE 3D PLOTTING ~S28

**Description**

[Technical Field]

This application claims priority to and the benefit of Korean Patent Application No. 10-2021-0113915, filed on August 27, 2021, Korean Patent Application No. 10-2021-0138918, filed on October 19, 2021, and Korean Patent Application No. 10-2022-0086165, filed on July 13, 2022, the disclosures of which are incorporated herein by reference in their entireties.

The present invention relates to a system and method for predicting the physical properties of a multilayer material, and more particularly, to a system and method for predicting the physical properties of a multilayer material when developing the multilayer material.

[Background Art]

A polymer film refers to a non-fibrous flat plastic molded article, which is light, has a good barrier property, high transparency and is relatively inexpensive, so it is used in many fields such as packaging materials, household goods, electronic devices, automobiles, and aircraft.

Synthetic polymers such as polyethylene (PE), polypropylene (PP), polyvinyl chloride (PVC), and polyethylene terephthalate (PET) are processed into a polymer film and widely used in Korea and abroad, and currently, many synthetic polymers are used as materials for polymer films either alone or by blending.

A multilayer film is a composite film in which different types of films are laminated for the purpose of multi-functionality of the film, and various types of multilayer films formed of, for example, the combination of a film having the excellent mechanical property of polyethylene (PE) and the printing aesthetics of cellophane, and nylon and a vinyl alcohol-ethylene copolymer are used as packaging materials.

In the case of developing such a multilayer film as a material, it is necessary to predict a physical property such as an expansion coefficient of an entire laminate.

The expansion coefficient is a value indicating the rate of increase in length or volume of a material when the same is heated or absorbs water. The former is a coefficient of thermal expansion, and the latter is a coefficient of water expansion.

Since multilayer materials show anisotropy in a machine direction (MD) and a transverse direction (TD) in a manufacturing process, for the design of a robust material, it is necessary to predict not only the homogenized stiffness of the entire laminate of a multilayer material, but also warpage that is undesirably generated due to the asymmetric structure caused by thermal and water expansions.

In the process of developing a multilayer material, the homogenized coefficients of thermal expansion and water expansion of the entire laminate are directly related to the deformation of the final product. Therefore, in order to design a robust material, it is necessary to predict warpage as well as thermal expansion and water expansion considering environmental factors such as a temperature change and a humidity change.

[Disclosure]

[Technical Problem]

To solve technical problems of the present invention, the present invention is directed to providing a system and method for predicting the physical properties of a multilayer material, which can predict physical properties such as thermal and coefficients of water expansion of an entire laminate when a multilayer material is developed, and predict warpage.

[Technical Solution]

In one embodiment for accomplishing the above-described purpose, a system for predicting the physical properties of a multilayer material having n laminated films (n is an integer of 2 or more) according to the present invention includes:

an input unit to which input values including any one or more of an elastic modulus ($E^k$) of each layer (k), a Poisson's ratio ($\upsilon^k$) of each layer (k), a shear modulus ($G^k$) of each layer (k), a thickness ($Z^k$) of each layer (k), and a lamination angle ($\theta^k$) of each layer (k), coefficients of thermal expansion ($\alpha^k_{1,2}$) or coefficients of water expansion ($\beta^k_{1,2}$) of each layer (k), a temperature change ($\Delta T$), or a humidity change ($\Delta C$) are input;
a control unit that calculates the physical properties of the multilayer material by applying input values to the input unit;

a display that is connected to the control unit; and
a storage unit that is connected to the control unit.

In addition, the present invention provides a method of predicting the physical properties of a multilayer material. In one embodiment, the method of predicting the physical properties of a multilayer material having n laminated films (n is an integer of 2 or more) according to the present invention includes inputting input values including any one or more of an elastic modulus ($E^k$) of each layer (k), a Poisson's ratio ($\upsilon^k$) of each layer (k), a shear modulus ($G^k$) of each layer (k), a thickness ($Z^k$) of each layer (k), or a lamination angle ($\theta^k$) of each layer (k), coefficients of thermal expansion ($\alpha^k_{1,2}$) or coefficients of water expansion ($\beta^k_{1,2}$) of each layer (k), a temperature change ($\Delta T$), or a humidity change ($\Delta C$); and calculating one or more output values of a coefficient of thermal expansion ($\alpha$) of the multilayer material, a coefficient of water expansion ($\beta$) of the multilayer material, or a warpage of the multilayer material by applying the input values.

[Advantageous Effects]

The present invention can predict physical properties such as a coefficient of thermal expansion and a coefficient of water expansion of an entire laminate when developing a multilayer material, and predict warpage.

[Brief Description of the Drawings]

FIG. 1 is a diagram illustrating a configuration of a system for predicting the physical properties of a multilayer material according to a first embodiment of the present invention.
FIG. 2 is a diagram illustrating a configuration of an input screen of the system for predicting the physical properties of a multilayer material according to the first embodiment of the present invention.
FIG. 3 is a diagram illustrating a configuration of an output screen of the system for predicting the physical properties of a multilayer material according to the first embodiment of the present invention.
FIG. 4 is a flowchart of a method of predicting the physical properties of a multilayer material according to a second embodiment of the present invention.
FIG. 5 is a flowchart of a method of predicting the physical properties of a multilayer material according to a third embodiment of the present invention.

[Detailed Description of the Preferred Embodiments]

In one embodiment for achieving the above-described purposes, a system for predicting the physical properties of a multilayer material having n laminated films (n is an integer of 2 or more) includes an input unit to which input values including any one or more of an elastic modulus ($E^k$) of each layer (k), a Poisson's ratio ($\upsilon^k$) of each layer (k), a shear modulus ($G^k$) of each layer (k), a thickness ($Z^k$) of each layer (k), or a lamination angle ($\theta^k$) of each layer (k), coefficients of thermal expansion ($\alpha^k_{1,2}$) or coefficients of water expansion ($\beta^k_{1,2}$) of each layer (k), a temperature change ($\Delta T$), or a humidity change ($\Delta C$) are input;

a control unit that calculates the physical properties of the multilayer material by applying input values to the input unit;
a display that is connected to the control unit; and
a storage unit that is connected to the control unit.

In addition, the control unit processes the values input to the input unit to calculate any one or more of a coefficient of thermal expansion ($\alpha$) of the multilayer material, a coefficient of water expansion ($\beta$) of the multilayer material, or a warpage of the multilayer material.
In an exemplary embodiment, the values input to the input unit include any one or more of elastic moduli ($E^k_{1,2}$) in the machine direction (1) or the transverse direction (2) of each layer (k),
**[0018]** Poisson's ratios ($\upsilon^k_{1,2}$) in the machine direction (1) or the transverse direction (2) of each layer (k),

shear moduli ($G^K_{1,2}$) in the machine direction (1) or the transverse direction (2) of each layer (k),
an angle ($\theta^k$) in the machine direction (1) of each layer with respect to the x direction of the multilayer material,
wherein the x direction means an arbitrarily set direction in a plane of the multilayer material, or
a thickness ($Z^k$) of each layer (k); or
any one or more of coefficients of thermal expansion ($\alpha^k_{1,2}$), coefficients of water expansion ($\beta^k_{1,2}$), a temperature change ($\Delta T$), or a humidity change ($\Delta C$) of each layer (k).

In an exemplary embodiment, to the input unit, elastic moduli ($E^k_{1,2}$) in the machine direction (MD, 1) and the transverse direction (TD, 2) of each layer (k), Poisson's ratios ($\upsilon^k_{1,2}$) in the machine direction (1) and the transverse direction (2) of each layer (k), shear moduli ($G^K_{1,2}$) in the machine direction (1) and the transverse direction (2) of each layer (k), an angle ($\theta^k$) in the machine direction (1) of each layer with respect to the x direction of the multilayer material, wherein the x direction means an arbitrarily set direction in a plane of the multilayer material, a thickness ($Z^k$) of each layer (k), coefficients of thermal expansion ($\alpha^k_{1,2}$) and coefficients of water expansion ($\beta^k_{1,2}$) of each layer (k), a temperature change ($\Delta T$), and a humidity change ($\Delta C$) are input.

In another exemplary embodiment, the control unit calculates stiffness matrices ($[Q]^k_{1,2}$) in the machine direction (1) and the transverse direction (2) of each layer (k) using the elastic moduli ($E^k_{1,2}$), Poisson's ratios ($\upsilon^k_{1,2}$), and shear moduli ($G^k_{1,2}$),

sets inverse matrices ($[S]^k_{1,2}$) for the stiffness matrices ($[Q]^k_{1,2}$) in the machine direction (1) and the transverse direction (2) of each layer (k),

resets stiffness matrices ($[Q]^k_{x,y}$) of each layer (k) by reflecting a lamination angle ($\theta^k$) of the multilayer material in the stiffness matrices ($[Q]^k_{1,2}$),

calculates stiffness matrices ($[A]_{x,y}$, $[B]_{x,y}$, $[D]_{x,y}$) of the multilayer material using the values of the reset stiffness matrices by receiving the thickness information of each layer (k),

sets compliance matrices ($[a]_{x,y}$, $[b]_{x,y}$, $[c]_{x,y}$, $[d]_{x,y}$) for the stiffness matrices ($[A]_{x,y}$, $[B]_{x,y}$, $[D]_{x,y}$) of the multilayer material,

calculates free lamina hydrothermal strains ($e^k_{1,2}$) generated by water expansion of each layer (k) in the major direction of each layer (k) using the coefficients of thermal expansion ($\alpha^k_{1,2}$), coefficients of water expansion ($\beta^k_{1,2}$), temperature change ($\Delta T$), and humidity change ($\Delta C$) of each layer (k),

calculates hygrothermal strain transformations ($e^k_{x,y,s}$) of each layer (k) by reflecting a lamination angle ($\theta^k$) of the multilayer material in the free lamina hydrothermal strains ($e^k_{1,2}$),

calculates hygrothermal forces ($N^{HT}_{x,y,s}$) and hygrothermal moments ($M^{HT}_{x,y,s}$), generated in the multilayer material, based on the hygrothermal strain transformations ($e^k_{x,y,s}$) of the multilayer material, the stiffness matrices ($[Q]^k_{x,y}$) of the multilayer material, which is the entire laminate, and the thickness ($Z^k$) of each layer (k),

forms total forces (/N) and total moments (/M) by adding external forces (N, M) to the hygrothermal forces ($N^{HT}_{x,y,s}$) and the hygrothermal moments ($M^{HT}_{x,y,s}$), and

calculates a coefficient of thermal expansion ($\alpha$) and coefficient of water expansion ($\beta$) of the multilayer material using the total forces (/N) and the total moments (/M), and the compliance matrices ($[a]_{x,y}$, $[b]_{x,y}$, $[c]_{x,y}$, $[d]_{x,y}$) for the stiffness matrices ($[A]_{x,y}$, $[B]_{x,y}$, $[D]_{x,y}$) of the multilayer material.

In still another embodiment, the control unit calculates strains ($\varepsilon^0_{x,y}$) and curvatures ($k_{x,y,s}$) of a middle plane using the total forces (/N) and the total moments (/M), and the compliance matrices ($[a]_{x,y}$, $[b]_{x,y}$, $[c]_{x,y}$, $[d]_{x,y}$) for the stiffness matrices ($[A]_{x,y}$, $[B]_{x,y}$, $[D]_{x,y}$) of the multilayer material, and calculates a warpage of the multilayer material by utilizing the curvatures ($k_{x,y,s}$) of the middle plane, and sample size (x, y) information.

In yet another embodiment, the control unit also calculates elastic moduli ($E_{x,y}$), shear moduli ($G_{x,y}$), and Poisson's ratios ($\upsilon_{x,y}$) of the multilayer material using the total thickness (h) of the multilayer material and the values of the compliance matrices ($[a]_{x,y}$, $[b]_{x,y}$, $[c]_{x,y}$, $[d]_{x,y}$) .

In one exemplary embodiment, to the input unit, elastic moduli ($E^k_{1,2}$) in the machine direction (MD, 1) and the transverse direction (TD, 2) of each layer (k), Poisson's ratios ($\upsilon^k_{1,2}$) in the machine direction (1) and the transverse direction (2) of each layer (k), shear moduli ($G^K_{1,2}$) in the machine direction (1) and the transverse direction (2) of each layer (k), an angle ($\theta^k$) in the machine direction (1) of each layer with respect to the x direction of the multilayer material, wherein the x direction means an arbitrarily set direction in a plane of the multilayer material, a thickness ($Z^k$) of each layer (k), coefficients of thermal expansion ($\alpha^k_{1,2}$) and coefficients of water expansion ($\beta^k_{1,2}$) of each layer (k), a temperature change ($\Delta T$), and a humidity change ($\Delta C$) are input.

In yet another exemplary embodiment, the control unit calculates stiffness matrices ($[Q]^k_{1,2}$) in the machine direction (1) and the transverse direction (2) of each layer (k) using the elastic moduli ($E^k_{1,2}$), Poisson's ratios ($\upsilon^k_{1,2}$), and shear moduli ($G^k_{1,2}$),

sets inverse matrices ($[S]^k_{1,2}$) for the stiffness matrices ($[Q]^k_{1,2}$) in the machine direction (1) and the transverse direction (2) of each layer (k),

resets stiffness matrices ($[Q]^k_{x,y}$) of each layer (k) by reflecting a lamination angle ($\theta^k$) of the multilayer material in the stiffness matrices ($[Q]^k_{1,2}$),

calculates stiffness matrices ($[A]_{x,y}$, $[B]_{x,y}$, $[D]_{x,y}$) of the multilayer material using the values of the reset stiffness matrices by receiving the thickness information of each layer (k),

sets compliance matrices ($[a]_{x,y}$, $[b]_{x,y}$, $[c]_{x,y}$,$[d]_{x,y}$) for the stiffness matrices ($[A]_{x,y}$, $[B]_{x,y}$, $[D]_{x,y}$) of the multilayer material,

calculates elastic moduli ($E_{x,y}$), shear moduli ($G_{x,y}$), and Poisson's ratios ($\upsilon_{x,y}$) of the multilayer material using the total thickness (h) of the multilayer material and the values of the compliance matrices ($[a]_{x,y}$, $[b]_{x,y}$, $[c]_{x,y}$,$[d]_{x,y}$),

calculates free lamina hydrothermal strains ($e^k_{1,2}$) generated by water expansion of each layer (k) in the major direction of each layer (k) using the coefficients of thermal expansion ($\alpha^k_{1,2}$), coefficients of water expansion ($\beta^k_{1,2}$), temperature change ($\Delta T$), and humidity change ($\Delta C$) of each layer (k),

calculates hygrothermal strain transformations ($e^k_{x,y,s}$) of each layer (k) by reflecting a lamination angle ($\theta^k$) of each layer (k) in the free lamina hydrothermal strains,

calculates hygrothermal forces ($N^{HT}_{x,y,s}$) and hygrothermal moments ($M^{HT}_{x,y,s}$) generated in the multilayer material based on the hygrothermal strain transformations ($e^k_{x,y,s}$) of each layer (k), the stiffness matrices ($[Q]^k_{x,y}$) of each layer (k), and the thickness ($Z^k$) of each layer (k),

forms total forces (/N) and total moments (/M) by adding external forces (N, M) to the hygrothermal forces ($N^{HT}_{x,y,s}$) and the hygrothermal moments ($M^{HT}_{x,y,s}$),

calculates a coefficient of thermal expansion ($\alpha$) and coefficient of water expansion ($\beta$) of the multilayer material using the total forces (/N) and the total moments (/M), and the compliance matrices ($[a]_{x,y}$, $[b]_{x,y}$, $[c]_{x,y}$,$[d]_{x,y}$) for the stiffness matrices ($[A]_{x,y}$, $[B]_{x,y}$, $[D]_{x,y}$) of the multilayer material,

calculates strains ($\varepsilon^0_{x,y}$) and curvatures ($k_{x,y,s}$) of a middle plane using the total forces (/N) and the total moments (/M), and the compliance matrices ($[a]_{x,y}$, $[b]_{x,y}$, $[c]_{x,y}$,$[d]_{x,y}$) for the stiffness matrices ($[A]_{x,y}$, $[B]_{x,y}$, $[D]_{x,y}$) of the multilayer material, and

calculates a warpage of the multilayer material by utilizing the curvature ($k_{x,y,s}$) of the middle plane, and sample size (x, y) information.

In addition, the present invention provides a method of predicting the physical properties of a multilayer material. In one embodiment, the method of predicting the physical properties of a multilayer material having n laminated films (n is an integer of 2 or more), includes

inputting input values including any one or more of an elastic modulus ($E^k$) of each layer (k), a Poisson's ratio ($\upsilon^k$) of each layer (k), a shear modulus ($G^k$) of each layer (k), a thickness ($Z^k$) of each layer (k), or a lamination angle ($\theta^k$) of each layer (k), coefficients of thermal expansion ($\alpha^k_{1,2}$) or coefficients of water expansion ($\beta^k_{1,2}$) of each layer (k), a temperature change ($\Delta T$), or a humidity change ($\Delta C$); and

calculating any one or more output values of a coefficient of thermal expansion ($\alpha$) of the multilayer material, a coefficient of water expansion ($\beta$) of the multilayer material, or a warpage of the multilayer material by applying the input values.

In one embodiment, in the inputting of input values, the input values include any one or more of elastic moduli ($E^k_{1,2}$) in the machine direction (1) or transverse direction (2) of each layer (k),

Poisson's ratios ($\upsilon^k_{1,2}$) in the machine direction (1) or transverse direction (2) of each layer (k),

shear moduli ($G^K_{1,2}$) in the machine direction (1) or transverse direction (2) of each layer (k),

an angle ($\theta^k$) in the machine direction (1) of each layer with respect to the x direction of the multilayer material, wherein the x direction means an arbitrarily set direction in a plane of the multilayer material, or

a thickness ($Z^k$) of each layer (k); or

any one or more of coefficients of thermal expansion ($\alpha^k_{1,2}$), coefficients of water expansion ($\beta^k_{1,2}$), a temperature change ($\Delta T$), or a humidity change ($\Delta C$) of each layer (k).

In one embodiment, in the method of predicting the physical properties of a multilayer material according to the present invention, the inputting of input values includes inputting elastic moduli ($E^k_{1,2}$) in the machine direction (1) and the transverse direction (2) of each layer (k), Poisson's ratios ($\upsilon^k_{1,2}$) in the machine direction (1) or the transverse direction (2) of each layer (k), shear moduli ($G^K_{1,2}$) in the machine direction (1) or the transverse direction (2) of each layer (k), an angle ($\theta^k$) in the machine direction (1) of each layer (k) with respect to the x direction of the multilayer material, and a thickness ($Z^k$) of each layer (k) (S11).

In another embodiment, in the method of predicting the physical properties of a multilayer material according to the present invention, the calculating of output values includes calculating stiffness matrices ($[Q]^k_{1,2}$) in the machine direction (1) and the transverse direction (2) of each layer (k) using the elastic moduli ($E^k_{1,2}$), Poisson's ratios ($\upsilon^k_{1,2}$), and shear moduli ($G^k_{1,2}$) (S12);

setting inverse matrices ($[S]^k_{1,2}$) for the stiffness matrices ($[Q]^k_{1,2}$) in the machine direction (1) and the transverse direction (2) of each layer (k) (S13);

resetting stiffness matrices ($[Q]^k_{x,y}$) of the multilayer material by reflecting a lamination angle ($\theta^k$) of each layer (k) in the stiffness matrices ($[Q]^k_{1,2}$) (S14);

calculating stiffness matrices ($[A]_{x,y}$, $[B]_{x,y}$, $[D]_{x,y}$) of the multilayer material using the values of the reset stiffness matrices by receiving the thickness information of each layer (k) (S15);

setting compliance matrices ($[a]_{x,y}$, $[b]_{x,y}$, $[c]_{x,y}$, $[d]_{x,y}$) for the stiffness matrices ($[A]_{x,y}$, $[B]_{x,y}$, $[D]_{x,y}$) of the multilayer material (S16);

inputting coefficients of thermal expansion ($\alpha^k_{1,2}$), coefficients of water expansion ($\beta^k_{1,2}$), a temperature change ($\Delta T$), and a humidity change ($\Delta C$) of each layer (k) (S21);

calculating free lamina hydrothermal strains ($e^k_{1,2}$) generated by water expansion of each layer (k) in the major direction of each layer (k) using the coefficients of thermal expansion ($\alpha^k_{1,2}$), coefficients of water expansion ($\beta^k_{1,2}$), temperature change ($\Delta T$), and humidity change ($\Delta C$) of each layer (k) (S22);

calculating hygrothermal strain transformations ($e^k_{x,y,s}$) of the multilayer material by reflecting a lamination angle ($\theta^k$) of the multilayer material in the free lamina hydrothermal strains ($e^k_{1,2}$) (S23);

calculating hygrothermal forces ($N^{HT}_{x,y,s}$) and hygrothermal moments ($M^{HT}_{x,y,s}$), generated in the multilayer material, based on the hygrothermal strain transformations ($e^k_{x,y,s}$) of the multilayer material, the stiffness matrices ($[Q]^k_{x,y}$) of the multilayer material, which is the entire laminate, and the thickness ($Z^k$) of each layer (k) (S24);

forming total forces ($/N$) and total moments ($/M$) by adding external forces (N, M) to the hygrothermal forces ($N^{HT}_{x,y,s}$) and the hygrothermal moments ($M^{HT}_{x,y,s}$) (S25); and

calculating a coefficient of thermal expansion ($\alpha$) and coefficient of water expansion ($\beta$) of the multilayer material using the total forces ($/N$) and the total moments ($/M$), and the compliance matrices ($[a]_{x,y}$, $[b]_{x,y}$, $[c]_{x,y}$, $[d]_{x,y}$) for the stiffness matrices ($[A]_{x,y}$, $[B]_{x,y}$, $[D]_{x,y}$) of the multilayer material (S26).

In still another embodiment, the method of predicting the physical properties of a multilayer material according to the present invention includes calculating strains ($\varepsilon^0_{x,y}$) and curvatures ($k_{x,y,s}$) of a middle plane using the total forces ($/N$) and the total moments ($/M$), and the compliance matrices ($[a]_{x,y}$, $[b]_{x,y}$, $[c]_{x,y}$, $[d]_{x,y}$) for the stiffness matrices ($[A]_{x,y}$, $[B]_{x,y}$, $[D]_{x,y}$) of the multilayer material (S27); and

calculating a warpage of the multilayer material by utilizing the curvatures ($k_{x,y,s}$) of the middle plane, and sample size (x, y) information (S28).

In yet another embodiment, the method of predicting the physical properties of a multilayer material according to the present invention further includes, after the setting of the compliance matrices ($[a]_{x,y}$, $[b]_{x,y}$, $[c]_{x,y}$, $[d]_{x,y}$) (S16), calculating elastic moduli ($E_{x,y}$), shear moduli ($G_{x,y}$), and Poisson's ratios ($\upsilon_{x,y}$) of the multilayer material using the total thickness (h) of the multilayer material and the values of the compliance matrices ($[a]_{x,y}$, $[b]_{x,y}$, $[c]_{x,y}$, $[d]_{x,y}$) (S17).

In an exemplary embodiment, in the method of predicting the physical properties of a multilayer material according to the present invention, the inputting of input values includes inputting elastic moduli ($E^k_{1,2}$) in the machine direction (1) and the transverse direction (2) of each layer (k), Poisson's ratios ($\upsilon^k_{1,2}$) in the machine direction (1) and the transverse direction (2) of each layer (k), shear moduli ($G^k_{1,2}$) in the machine direction (1) and the transverse direction (2) of each layer (k), an angle ($\theta^k$) in the machine direction (1) of each layer (k) with respect to the x direction of the multilayer material, and a thickness ($Z^k$) of each layer (k) (S11).

In another embodiment, in the method of predicting the physical properties of a multilayer material according to the present invention, the calculating of output values includes calculating stiffness matrices ($[Q]^k_{1,2}$) in the machine direction (1) and the transverse direction (2) of each layer (k) using the elastic moduli ($E^k_{1,2}$), Poisson's ratios ($\upsilon^k_{1,2}$), and shear moduli ($G^k_{1,2}$) (S12);

setting inverse matrices ($[S]^k_{1,2}$) for the stiffness matrices ($[Q]^k_{1,2}$) in the machine direction (1) and the transverse direction (2) of each layer (k) (S13);

resetting stiffness matrices ($[Q]^k_{x,y}$) of the multilayer material by reflecting a lamination angle ($\theta^k$) of each layer (k) in the stiffness matrices ($[Q]^k_{1,2}$) (S14);

calculating stiffness matrices ($[A]_{x,y}$, $[B]_{x,y}$, $[D]_{x,y}$) of the multilayer material using the values of the reset stiffness matrices by receiving the thickness information of each layer (k) (S15);

setting compliance matrices ($[a]_{x,y}$, $[b]_{x,y}$, $[c]_{x,y}$, $[d]_{x,y}$) for the stiffness matrices ($[A]_{x,y}$, $[B]_{x,y}$, $[D]_{x,y}$) of the multilayer material (S16);

calculating elastic moduli ($E_{x,y}$), shear moduli ($G_{x,y}$), and Poisson's ratios ($\upsilon_{x,y}$) of the multilayer material using the total thickness (h) of the multilayer material and values of the compliance matrices ($[a]_{x,y}$, $[b]_{x,y}$, $[c]_{x,y}$, $[d]_{x,y}$) (S17);

inputting coefficients of thermal expansion ($\alpha^k_{1,2}$), coefficients of water expansion ($\beta^k_{1,2}$), a temperature change ($\Delta T$), and a humidity change ($\Delta C$) of each layer (k) (S21);

calculating free lamina hydrothermal strains ($e^k_{1,2}$) generated by water expansion of each layer (k) in the major direction of each layer (k) using the coefficients of thermal expansion ($\alpha^k_{1,2}$), coefficients of water expansion ($\beta^k_{1,2}$), temperature change ($\Delta T$), and humidity change ($\Delta C$) of each layer (k) (S22);

calculating hygrothermal strain transformations ($e^k_{x,y,s}$) of the multilayer material by reflecting a lamination angle ($\theta^k$) of the multilayer material in the free lamina hydrothermal strains ($e^k_{1,2}$) (S23);

calculating hygrothermal forces ($N^{HT}_{x,y,s}$) and hygrothermal moments ($M^{HT}_{x,y,s}$), generated in the multilayer material, based on the hygrothermal strain transformations ($e^k_{x,y,s}$) of the multilayer material, the stiffness matrices ($[Q]^k_{x,y}$) of the multilayer material, which is the entire laminate, and the thickness ($Z^k$) of each layer (k) (S24);

forming total forces (/N) and total moments (/M) by adding external forces (N, M) to the hygrothermal forces ($N^{HT}_{x,y,s}$) and the hygrothermal moments ($M^{HT}_{x,y,s}$) (S25);

calculating a coefficient of thermal expansion ($\alpha$) and coefficient of water expansion ($\beta$) of the multilayer material using the total forces (/N) and the total moments (/M), and the compliance matrices ($[a]_{x,y}$, $[b]_{x,y}$, $[c]_{x,y}$, $[d]_{x,y}$) for the stiffness matrices ($[A]_{x,y}$, $[B]_{x,y}$, $[D]_{x,y}$) of the multilayer material (S26);

calculating calculates strains ($\varepsilon^0_{x,y}$) and curvatures ($k_{x,y,s}$) of a middle plane using the total forces (/N) and the total moments (/M), and the compliance matrices ($[a]_{x,y}$, $[b]_{x,y}$, $[c]_{x,y}$, $[d]_{x,y}$) for the stiffness matrices ($[A]_{x,y}$, $[B]_{x,y}$, $[D]_{x,y}$) of the multilayer material (S27); and

calculating a warpage of the multilayer material by utilizing the curvature ($k_{x,y,s}$) of the middle plane, and sample size (x, y) information (S28).

Hereinafter, to describe the technical idea of the present invention in detail so as to be easily implemented by those of ordinary skill in the art to which the present invention belongs, preferred embodiments will be described with reference to the accompanying drawings. Other objects, features, and operational advantages of the present invention, including the object, action, and effect, will be more obvious by the description of preferred embodiments.

For reference, the embodiments disclosed herein are only presented by selecting and presenting the most preferred embodiments to help understanding of those of ordinary skill in the art from among various possible embodiments, and the technical spirit of the present invention is not necessarily limited by the presented embodiments. Various changes, additions, and modifications including equivalents or substitutes are possible without departing from the technical spirit of the present invention.

In addition, terms or words used in in the specification and claims are defined based on the principle in that an inventor can be adequately define the concept of a term in order to describe his/her invention in the best way, and should not be construed as being limited only to the ordinary or dictionary meanings, but should be interpreted with meanings and concepts consistent with the technical spirit of the present invention. As an example, singular expressions include plural expressions unless clearly indicated otherwise in the context, expressions regarding the direction are set based on the position expressed on the drawings for convenience of description, and the expression "connect" or "access" includes not only direct connection or access but also connection or access through a different component between two components. In addition, the expression "unit" includes a unit implemented using hardware, a unit implemented using software, a unit implemented using both hardware and software, and one unit may be implemented using one or more pieces of hardware or software, and two or more units may be implemented using one piece of hardware or software.

**(First embodiment)**

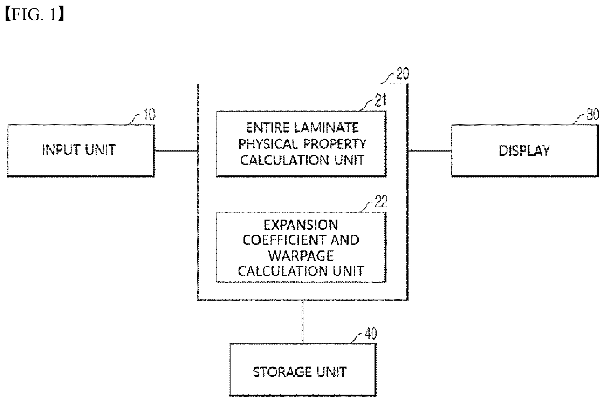

FIG. 1 is a diagram illustrating a configuration of a system for predicting the physical properties of a multilayer material according to a first embodiment of the present invention.

As illustrated in FIG. 1, the configuration of the system for predicting the physical properties of a multilayer material according to the first embodiment of the present invention includes an input unit 10 for inputting elastic moduli ($E^k_{1,2}$) in a machine direction (MD, hereinafter, set as ' 1,' denoting a major direction) and a transverse direction (TD, hereinafter, set as '2') of each layer (k), Poisson's ratios ($\upsilon^k_{1,2}$) in the machine direction (1) and transverse direction (2) of each layer (k), shear moduli ($G^K_{1,2}$) in the machine direction (1) and transverse direction (2) of each layer (k), an angle ($\theta^k$) in the machine direction (1) of each layer with respect to the x direction of the multilayer material, a thickness ($Z^k$) of each layer (k), coefficients of thermal expansion ($\alpha^k_{1,2}$) in the machine direction (1) and transverse direction (2) of each layer (k), coefficients of water expansion ($\beta^k_{1,2}$) in the machine direction (1) and the transverse direction (2) of each layer (k), a temperature change ($\Delta T$), and a humidity change ($\Delta C$) by a user, a control unit 20, which is connected to the input unit 10 and includes an entire laminate physical property calculation unit 21 and an expansion coefficient and warpage calculation unit 22, a display 30 connected to the control unit 20, and a storage unit 40 connected to the control unit 20.

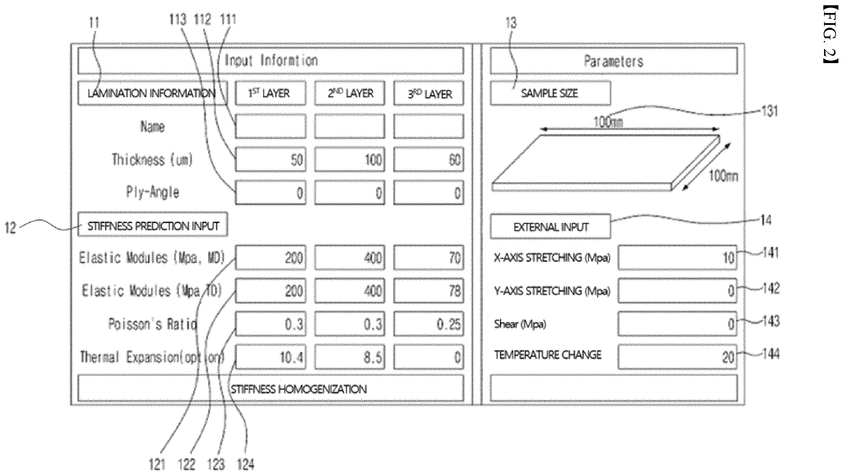

FIG. 2 is a diagram illustrating a configuration of an input screen of the system for predicting the physical properties of a multilayer material according to the first embodiment of the present invention.

As shown in FIG. 2, the configuration of an input screen of the system for predicting the physical properties of a multilayer material according to one embodiment of the present invention is composed of a lamination information input unit 11 in which a name input field 111, a thickness input field 112, and an angle input field 113 are arranged for each layer; a stiffness prediction input unit 12 in which a machine direction (MD) elastic modulus input field 121, a transverse direction (TD) elastic modulus input field 122, a Poisson's ratio input field 123, and a thermal expansion rate input field 124 are arranged for each layer; a sample size input unit 13 in which a sample width and length input field 131 is arranged; and an external force input unit 14 in which an X-axis tensile force input field 141, a Y-axis tensile force input field 142, a shear force input field 143, and a temperature change input field 144 are arranged.

In the case of the input screen of the first embodiment of the present invention, although the multilayer material is set to have three film layers, an input screen having 4 or more film layers may be provided.

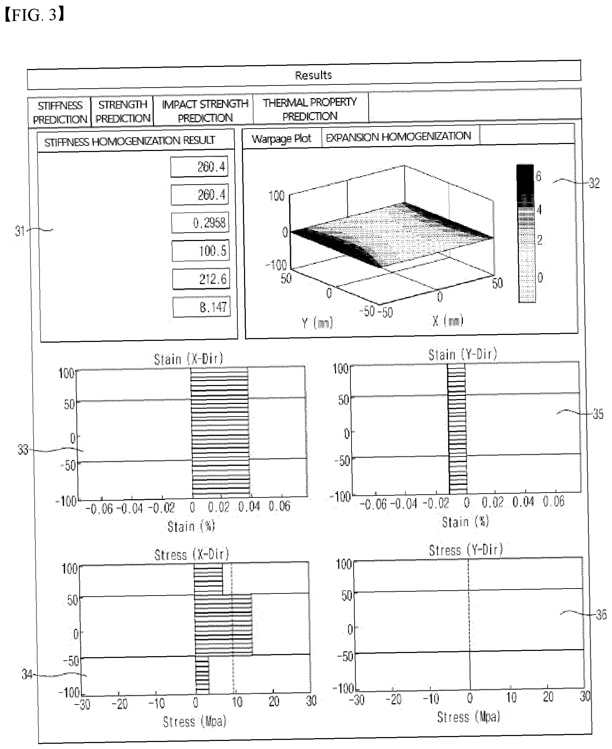

FIG. 3 is a diagram illustrating a configuration of an output screen of the system for predicting the physical properties of a multilayer material according to the first embodiment of the present invention.

As shown in FIG. 3, an output screen of the system for predicting the physical properties of a multilayer material according to the first embodiment of the present invention, as stiffness homogenization results 31, outputs a machine direction (MD) elastic modulus, a transverse (TD) elastic modulus, a Poisson's ratio, a shear modulus, a bulk modulus, and a thermal expansion rate, outputs a warpage plot 32, and outputs a thickness-dependent x-direction strain 33, a thickness-dependent x-direction stress 34, a thickness-dependent y-direction strain 35, and a thickness-dependent y-direction stress 36.

**(Second embodiment)**

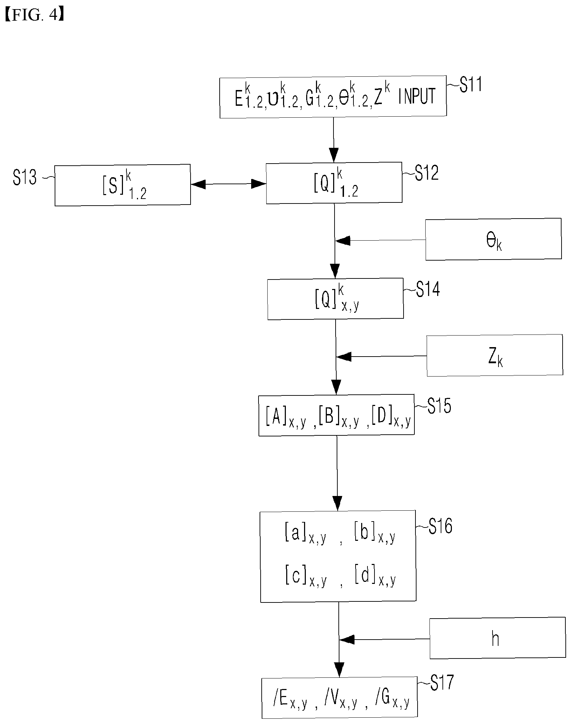

FIG. 4 is a flowchart of a method of predicting the physical properties of a multilayer material according to a second embodiment of the present invention. As shown in FIG. 4, the method of predicting the physical properties of a multilayer material according to the second embodiment of the present invention can be performed as follows.

For the multilayer material in which two or more materials are laminated, elastic moduli ($E^k_{1,2}$) in the machine direction (1) and the transverse direction (2) of each layer (k), Poisson's ratios ($\upsilon^k_{1,2}$) in the machine direction (1) and the transverse direction (2) of each layer (k), shear moduli ($G^K_{1,2}$) in the machine direction (1) and the transverse direction (2) of each layer (k), an angle ($\theta^k$) in the machine direction (1) of each layer (k) with respect to the x direction of the multilayer material, and a thickness ($Z^k$) of each layer (k) are input (S11).

Stiffness matrices ($[Q]^k_{1,2}$) in the machine direction (1) and the transverse direction (2) of each layer (k) are calculated using the elastic moduli ($E^k_{1,2}$), Poisson's ratios ($\upsilon^k_{1,2}$), and shear moduli ($G^k_{1,2}$) as shown in Equation 1 below (S12).

$$\begin{bmatrix} \sigma_1 \\ \sigma_2 \\ \tau_6 \end{bmatrix} = \begin{bmatrix} Q_{11} & Q_{12} & 0 \\ Q_{12} & Q_{22} & 0 \\ 0 & 0 & Q_{66} \end{bmatrix} \begin{bmatrix} \varepsilon_1 \\ \varepsilon_2 \\ \gamma_6 \end{bmatrix}$$

$$Q_{11} = \frac{E_1}{1 - V_{12} V_{21}} \qquad Q_{22} = \frac{E_2}{1 - V_{12} V_{21}}$$

$$Q_{12} = Q_{21} = \frac{V_{21} E_1}{1 - V_{12} V_{21}} = \frac{V_{12} E_2}{1 - V_{12} V_{21}}$$

$$Q_{66} = G_{12}$$

$$\tag{1}$$

(For an isotropic material, G = E / 2(1+$\upsilon$))

Inverse matrices ($[S]^k_{1,2}$) for the stiffness matrices ($[Q]^k_{1,2}$) in the machine direction (1) and the transverse direction (2) of each layer (k) that have been calculated as described above are set (S13).

Stiffness matrices ($[Q]^k_{x,y}$) of the multilayer material are reset by reflecting a lamination angle ($\theta^k$) of each layer (k) in the obtained stiffness matrices ($[Q]^k_{1,2}$) as shown in Equation (2) below (S14).

$$[T] = \begin{bmatrix} m^2 & n^2 & 2mn \\ n^2 & m^2 & -2mn \\ -mn & mn & m^2 - n^2 \end{bmatrix} \quad m=\cos\theta, \; n=\sin\theta$$

$$\begin{bmatrix} Q_{xx} & Q_{xy} & 2Q_{xs} \\ Q_{xy} & Q_{yy} & 2Q_{ys} \\ Q_{xs} & Q_{ys} & 2Q_{ss} \end{bmatrix} = [T^{-1}] \begin{bmatrix} Q_{11} & Q_{12} & 0 \\ Q_{12} & Q_{22} & 0 \\ 0 & 0 & 2Q_{66} \end{bmatrix} [T]$$

$$\begin{bmatrix} \sigma_x \\ \sigma_y \\ \tau_s \end{bmatrix} = \begin{bmatrix} Q_{xx} & Q_{xy} & Q_{xs} \\ Q_{xy} & Q_{yy} & Q_{ys} \\ Q_{xs} & Q_{ys} & Q_{ss} \end{bmatrix} \begin{bmatrix} \varepsilon_x \\ \varepsilon_y \\ \gamma_s \end{bmatrix} \quad \begin{array}{l} \text{Stress-strain relation reflecting angle} \\ (\text{dir-x,y}) \end{array}$$

$$(2)$$

In addition, the stiffness matrices ($[A]_{x,y}$, $[B]_{x,y}$, $[D]_{x,y}$) of the multilayer material, which is the entire laminate, are calculated using the values of the reset stiffness matrices by receiving the thickness information of each layer (k), as shown in Equation (3) below (S15).

$$A_{ij} = \sum_{k=1}^{n} Q_{ij}^{k}(z_k - z_{k-1}) \quad B_{ij} = \frac{1}{2} \sum_{k=1}^{n} Q_{ij}^{k}(z_k^2 - z_{k-1}^2)$$

$$D_{ij} = \frac{1}{3} \sum_{k=1}^{n} Q_{ij}^{k}(z_k^3 - z_{k-1}^3)$$

$$(3)$$

In Equation (3), k indicates each layer, and the multilayer material is formed of a total of n layers, wherein n is an integer of 2 to 10.

The compliance matrices ($[a]_{x,y}$, $[b]_{x,y}$, $[c]_{x,y}$, $[d]_{x,y}$) for the stiffness matrices ($[A]_{x,y}$, $[B]_{x,y}$, $[D]_{x,y}$) of the multilayer material, which is the entire laminate, calculated as described above are set as shown in Equation (4) below (S16).

$$\begin{bmatrix} A_{xx} & A_{xy} & A_{xs} & B_{xx} & B_{xy} & B_{xs} \\ A_{yx} & A_{yy} & A_{ys} & B_{yx} & B_{yy} & B_{ys} \\ A_{sx} & A_{sy} & A_{ss} & B_{sx} & B_{sy} & B_{ss} \\ B_{xx} & B_{xy} & B_{xs} & D_{xx} & D_{xy} & D_{xs} \\ B_{yx} & B_{yy} & B_{ys} & D_{yx} & D_{yy} & D_{ys} \\ B_{sx} & B_{sy} & B_{ss} & D_{sx} & D_{sy} & D_{ss} \end{bmatrix}^{-1} = \begin{bmatrix} a_{xx} & a_{xy} & a_{xs} & b_{xx} & b_{xy} & b_{xs} \\ a_{yx} & a_{yy} & a_{ys} & b_{yx} & b_{yy} & b_{ys} \\ a_{sx} & a_{sy} & a_{ss} & b_{sx} & b_{sy} & b_{ss} \\ c_{xx} & c_{xy} & c_{xs} & d_{xx} & d_{xy} & d_{xs} \\ c_{yx} & c_{yy} & c_{ys} & d_{yx} & d_{yy} & d_{ys} \\ c_{sx} & c_{sy} & c_{ss} & d_{sx} & d_{sy} & d_{ss} \end{bmatrix}$$

$$(4)$$

If necessary, elastic moduli ($/E_{x,y}$), shear moduli ($/G_{x,y}$), and Poisson's ratios ($/\upsilon_{x,y}$) of the multilayer material, which is the entire laminate, may be calculated using the total thickness (h) of the multilayer material, which is the entire laminate, and the values of the set compliance matrices ($[a]_{x,y}$, $[b]_{x,y}$, $[c]_{x,y}$, $[d]_{x,y}$) as shown in Equation (5) below.

$$/E_x = \frac{1}{ha_{xx}} \qquad /E_y = \frac{1}{ha_{yy}} \qquad /G_{xy} = \frac{1}{ha_{ss}}$$

$$/V_{xy} = -\frac{a_{yx}}{a_{xx}} \qquad /V_{yx} = -\frac{a_{xy}}{a_{yy}}$$

$$(5)$$

In the inputting of the elastic moduli ($E^k_{1,2}$), Poisson's ratios ($\upsilon^k_{1,2}$), and shear moduli ($G^K_{1,2}$) of each layer (k) (S11), the shear moduli ($G^k_{1,2}$) are calculated by a control unit 20 using the following relation between the elastic modulus (E), the shear modulus (G), and the Poisson's ratio ($\upsilon$) of an isotropic material.

$$G = \frac{E}{2(1+v)}$$

**(Third embodiment)**

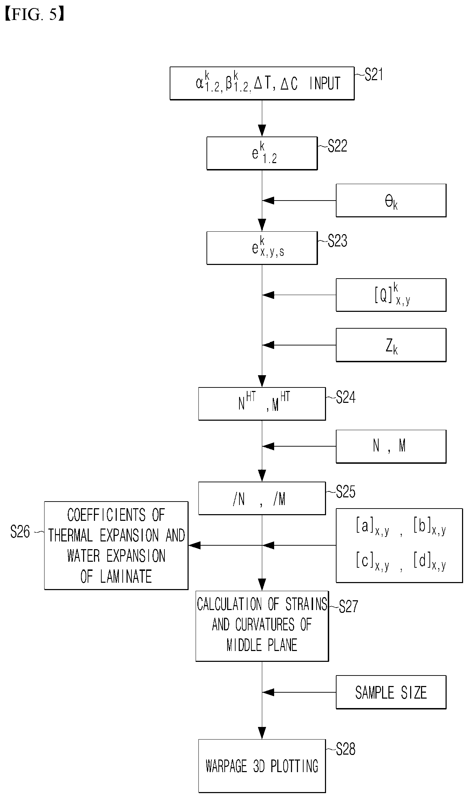

FIG. 5 is a flowchart of a method of predicting the physical properties of a multilayer material according to a third embodiment of the present invention. As shown in FIG. 5, the method of predicting the physical properties of a multilayer material according to a third embodiment of the present invention is as follows.

For the multilayer material in which two or more films are laminated, inputting coefficients of thermal expansion ($\alpha^k_{1,2}$), coefficients of water expansion ($\beta^k_{1,2}$), a temperature change ($\Delta T$), and a humidity change ($\Delta C$) of each layer (k) (S21) is performed.

Free lamina hydrothermal strains ($e^k_{1,2}$) generated by water expansion of each layer (k) in the major direction of each layer (k) are calculated using the input coefficients of thermal expansion ($\alpha^k_{1,2}$), coefficients of water expansion ($\beta^k_{1,2}$), temperature change ($\Delta T$), and humidity change ($\Delta C$) of each layer (k), as shown in Equation (6) below (S22).

$$e_1^k = \alpha_1^k \Delta T + \beta_1^k \Delta C \qquad\qquad e_2^k = \alpha_1^k \Delta T + \beta_1^k \Delta C \qquad (6)$$

Hygrothermal strain transformations ($e^k_{x,y,s}$) of the multilayer material, which is the entire laminate, are calculated by reflecting a lamination angle ($\theta^k$) of each layer (k) in the calculated free lamina hydrothermal strains, as shown in Equation (7) (S23).

$$e_x^k = e_1^k m^2 + e_2^k n^2 \qquad\qquad m = \cos\theta,\ n = \sin\theta$$

$$e_y^k = e_1^k n^2 + e_2^k m^2$$

$$e_s^k = 2(e_1^k + e_2^k) mn$$

$$(7)$$

Based on the hydrothermal strain transformations ($e^k_{x,y,s}$) of the multilayer material, which is the entire laminate, the stiffness matrices ($[Q]^k_{x,y}$) of the multilayer material, which is the entire laminate, calculated in the second embodiment, and the thickness ($Z^k$) of each layer (k), hygrothermal forces ($N^{HT}_{x,y,s}$) and hygrothermal moments ($M^{HT}_{x,y,s}$), generated in the multilayer material, which is the entire laminate, are calculated as shown in Equation (8) below (S24).

$$\begin{bmatrix} N^{HT}_x \\ N^{HT}_y \\ N^{HT}_s \end{bmatrix} = \sum_{k=1}^{n} \begin{bmatrix} Q_{xx} & Q_{xy} & Q_{xs} \\ Q_{xy} & Q_{yy} & Q_{ys} \\ Q_{xs} & Q_{ys} & Q_{ss} \end{bmatrix}_k \begin{bmatrix} e_x \\ e_y \\ e_s \end{bmatrix}_k t_k$$

$$\begin{bmatrix} M^{HT}_x \\ M^{HT}_y \\ M^{HT}_s \end{bmatrix} = \sum_{k=1}^{n} \begin{bmatrix} Q_{xx} & Q_{xy} & Q_{xs} \\ Q_{xy} & Q_{yy} & Q_{ys} \\ Q_{xs} & Q_{ys} & Q_{ss} \end{bmatrix}_k \begin{bmatrix} e_x \\ e_y \\ e_s \end{bmatrix}_k z_k t_k \qquad (8)$$

Total forces (/N) and total moments (/M) are formed by adding external forces (N, M), which are mechanical loads, to the hygrothermal forces ($N^{HT}_{x,y,s}$) and hygrothermal moments ($M^{HT}_{x,y,s}$) calculated above as shown in Equation (9) below (S25).

$$/N = N + N^{HT} \qquad /M = M + M^{HT} \qquad (9)$$

Using the total forces (/N) and the total moments (/M), and the compliance matrices ($[a]_{x,y}$, $[b]_{x,y}$, $[c]_{x,y}$, $[d]_{x,y}$) for the stiffness matrices ($[A]_{x,y}$, $[B]_{x,y}$, $[D]_{x,y}$) of the multilayer material, which is the entire laminate, calculated in the second embodiment, a coefficient of thermal expansion ($\alpha$) of the multilayer material, which is the entire laminate, is obtained as shown in Equation (10) below.

$$\begin{bmatrix} /\alpha_x \\ /\alpha_s \\ /\alpha_s \end{bmatrix} = \begin{bmatrix} \alpha_{xx} & \alpha_{xy} & \alpha_{xs} \\ \alpha_{yx} & \alpha_{yy} & \alpha_{ys} \\ \alpha_{sx} & \alpha_{sy} & \alpha_{ss} \end{bmatrix} \begin{bmatrix} N^T_x \\ N^T_y \\ N^T_s \end{bmatrix} + \begin{bmatrix} b_{xx} & b_{xy} & b_{xs} \\ b_{yx} & b_{yy} & b_{ys} \\ b_{sx} & b_{sy} & b_{ss} \end{bmatrix} \begin{bmatrix} M^T_x \\ M^T_y \\ M^T_s \end{bmatrix} \qquad (10)$$

(Here, provided that [N]=0, [M]=0, $\Delta T=1$)

A coefficient of water expansion ($\beta$) of the multilayer material, which is the entire laminate, is calculated as shown in Equation (11) below (S26).

$$w = -\frac{1}{2}(k_x x^2 + k_y y^2 + k_s xy) \qquad (11)$$

(Here, provided that [N]=0, [M]=0, $\Delta C=1$)

Using the total forces (/N) and the total moments (/M), and the compliance matrices ($[a]_{x,y}$, $[b]_{x,y}$, $[c]_{x,y}$, $[d]_{x,y}$) for the stiffness matrices ($[A]_{x,y}$, $[B]_{x,y}$, $[D]_{x,y}$) of the multilayer material, which is the entire laminate, calculated in the second embodiment, strains ($\varepsilon_{x,y}$) and curvatures ($k_{x,y,s}$) of a middle plane are calculated as shown in Equation (12) below (S27).

# EP 4 209 773 A1

$$\begin{bmatrix} \epsilon_x^0 \\ \epsilon_y^0 \\ \gamma_s^0 \\ \hline K_x \\ K_y \\ K_s \end{bmatrix} = \begin{bmatrix} a_{xx} & a_{xy} & a_{xs} & b_{xx} & b_{xy} & b_{xs} \\ a_{yx} & a_{yy} & a_{ys} & b_{yx} & b_{yy} & b_{ys} \\ a_{sx} & a_{sy} & a_{ss} & b_{sx} & b_{sy} & b_{ss} \\ \hline c_{xx} & c_{xy} & c_{xs} & d_{xx} & d_{xy} & d_{xs} \\ c_{yx} & c_{yy} & c_{ys} & d_{yx} & d_{yy} & d_{ys} \\ c_{sx} & c_{sy} & c_{ss} & d_{sx} & d_{sy} & d_{ss} \end{bmatrix} \begin{bmatrix} /N_x \\ /N_y \\ /N_s \\ \hline /M_x \\ /M_y \\ /M_s \end{bmatrix} \qquad (12)$$

A · · · B · · · C · · · D

A warpage is calculated as shown in Equation (13) and then 3D plotted by utilizing the curvatures of the middle plane, and the sample size (x, y) information input through the input unit 10 (S28).

$$w = -\frac{1}{2} \left( k_x x^2 + k_y y^2 + k_s xy \right) \qquad (13)$$

**(Fourth embodiment)**

The actions of the system and method for predicting the physical properties of a multilayer material according to the embodiments of the present invention, which have been configured as above, are as follows.

To predict the physical properties of the multilayer material, a user inputs elastic moduli ($E^k_{1,2}$) in the machine direction (1) and transverse direction (2) of each layer (k, k=1,2,3), Poisson's ratios ($\upsilon^k_{1,2}$) in the machine direction (1) and transverse direction (2) of each layer (k, k=1,2,3), shear moduli ($G^K_{1,2}$) in the machine direction (1) and transverse direction (2) of each layer (k, k=1,2,3), an angle ($\theta^k$) in the machine direction (1) of each layer (k, k=1,2,3) with respect to the x direction of the multilayer material, and a thickness ($Z^k$) of each layer (k) using an input unit 10 while looking at an input screen on a display 30 (S11).

On the input screen provided by the display 30, as shown in FIG. 2, a lamination data input unit 11 in which a name input field 111, a thickness input field 112, and an angle input field 113 are arranged for each layer, a stiffness prediction input unit 12 in which a machine direction (MD) elastic modulus input field 121, a transverse direction (TD) elastic modulus input field 122, a Poisson's ratio input field 123, and a thermal expansion rate input field 124 are arranged for each layer, a sample size input unit 13 in which a sample width and length input field 131 is arranged; and an external force input unit 14 in which an X-axis tensile force input field 141, a Y-axis tensile force input field 142, a shear force input field 143, and a temperature change input field 144 are arranged are provided, information that does not appear on the input screen is automatically calculated by the control unit 20 and stored in a storage unit 40. For example, when the machine direction (MD) elastic modulus ($E^k_1$), transverse direction (TD) elastic modulus ($E^k_2$), and Poisson's ratios ($\upsilon^k_{1,2}$) are input for each layer through the input screen and the input unit 10, a control unit 20 automatically calculates shear moduli ($G^k_{1,2}$) using the relationship between an elastic modulus (E), a shear modulus (G) and a Poisson's ratio ($\upsilon$) of an isotropic material below, and then stores the same in the storage unit 40.

$$G = \frac{E}{2(1+\upsilon)}$$

Next, an entire laminate physical property calculation unit 21 of the control unit 20 calculates stiffness matrices ($[Q]^k_{1,2}$) in the machine direction (1) and the transverse direction (2) of each layer (k) using the elastic moduli ($E^k_{1,2}$), Poisson's ratios ($\upsilon^k_{1,2}$), and shear moduli ($G^k_{1,2}$) as shown in Equation (1) below (S12).

$$\begin{bmatrix} \sigma_1 \\ \sigma_2 \\ \tau_6 \end{bmatrix} = \begin{bmatrix} Q_{11} & Q_{12} & 0 \\ Q_{12} & Q_{22} & 0 \\ 0 & 0 & Q_{66} \end{bmatrix} \begin{bmatrix} \varepsilon_1 \\ \varepsilon_2 \\ \gamma_6 \end{bmatrix}$$

$$Q_{11} = \frac{E_1}{1 - V_{12} V_{21}} \quad Q_{22} = \frac{E_2}{1 - V_{12} V_{21}}$$

$$Q_{12} = Q_{21} = \frac{V_{21} E_1}{1 - V_{12} V_{21}} = \frac{V_{12} E_2}{1 - V_{12} V_{21}}$$

$$Q_{66} = G_{12}$$

$$(1)$$

(The relation for an isotropic material G = E / 2(1+υ) is used)

Subsequently, the entire laminate physical property calculation unit 21 of the control unit 20 obtains inverse matrices ($[S]^k_{1,2}$) for the calculated stiffness matrices ($[Q]^k_{1,2}$) in the machine direction (1) and the transverse direction (2) of each layer (k), calculated above, and set as compliance matrices (S13). This is for transforming a stiffness matrix, which is a singular matrix, into an invertible matrix in which an inverse matrix is present.

Afterward, the entire laminate physical property calculation unit 21 of the control unit 20 resets stiffness matrices ($[Q]^k_{x,y}$) of each layer (k) by reflecting a lamination angle ($\theta^k$) of the multilayer material in the obtained stiffness matrices ($[Q]^k_{1,2}$) of each layer (k), as shown in Equation (2) below (S14).

$$[T] = \begin{bmatrix} m^2 & n^2 & 2mn \\ n^2 & m^2 & -2mn \\ -mn & mn & m^2 - n^2 \end{bmatrix} \quad m = \cos\theta, \ n = \sin\theta$$

$$\begin{bmatrix} Q_{xx} & Q_{xy} & 2Q_{xs} \\ Q_{xy} & Q_{yy} & 2Q_{ys} \\ Q_{xs} & Q_{ys} & 2Q_{ss} \end{bmatrix} = [T^{-1}] \begin{bmatrix} Q_{11} & Q_{12} & 0 \\ Q_{12} & Q_{22} & 0 \\ 0 & 0 & 2Q_{66} \end{bmatrix} [T]$$

$$\begin{bmatrix} \sigma_x \\ \sigma_y \\ \tau_s \end{bmatrix} = \begin{bmatrix} Q_{xx} & Q_{xy} & Q_{xs} \\ Q_{xy} & Q_{yy} & Q_{ys} \\ Q_{xs} & Q_{ys} & Q_{ss} \end{bmatrix} \begin{bmatrix} \varepsilon_x \\ \varepsilon_y \\ \gamma_s \end{bmatrix} \quad \text{Stress-strain relation reflecting angle} \atop (dir-x,y)$$

$$(2)$$

Subsequently, the entire laminate physical property calculation unit 21 of the control unit 20 calculates stiffness matrices ($[A]_{x,y}$, $[B]_{x,y}$, $[D]_{x,y}$) of the multilayer material, which is the entire laminate, using values of the reset stiffness matrices by receiving the thickness information of each layer (k), as shown in Equation (3) below (S15).

$$A_{ij} = \sum_{k=1}^{n} Q^k_{ij}(z_k - z_{k-1}) \quad B_{ij} = \frac{1}{2}\sum_{k=1}^{n} Q^k_{ij}(z_k^2 - z_{k-1}^2)$$

$$D_{ij} = \frac{1}{3}\sum_{k=1}^{n} Q^k_{ij}(z_k^3 - z_{k-1}^3)$$

$$(3)$$

In Equation (3), k indicates each layer, and the multilayer material is formed of a total of n layers, and n is an integer of 2 to 10.

Then, the entire laminate physical property calculation unit 21 of the control unit 20 sets compliance matrices ($[a]_{x,y}$, $[b]_{x,y}$, $[c]_{x,y}$, $[d]_{x,y}$) for the calculated stiffness matrices ($[A]_{x,y}$, $[B]_{x,y}$, $[D]_{x,y}$) of the multilayer material, which is the entire laminate, as shown in Equation (4) (S16).

$$
\begin{bmatrix}
A_{xx} & A_{xy} & A_{xs} & B_{xx} & B_{xy} & B_{xs} \\
A_{yx} & A_{yy} & A_{ys} & B_{yx} & B_{yy} & B_{ys} \\
A_{sx} & A_{sy} & A_{ss} & B_{sx} & B_{sy} & B_{ss} \\
B_{xx} & B_{xy} & B_{xs} & D_{xx} & D_{xy} & D_{xs} \\
B_{yx} & B_{yy} & B_{ys} & D_{yx} & D_{yy} & D_{ys} \\
B_{sx} & B_{sy} & B_{ss} & D_{sx} & D_{sy} & D_{ss}
\end{bmatrix}^{-1}
=
\begin{bmatrix}
a_{xx} & a_{xy} & a_{xs} & b_{xx} & b_{xy} & b_{xs} \\
a_{yx} & a_{yy} & a_{ys} & b_{yx} & b_{yy} & b_{ys} \\
a_{sx} & a_{sy} & a_{ss} & b_{sx} & b_{sy} & b_{ss} \\
c_{xx} & c_{xy} & c_{xs} & d_{xx} & d_{xy} & d_{xs} \\
c_{yx} & c_{yy} & c_{ys} & d_{yx} & d_{yy} & d_{ys} \\
c_{sx} & c_{sy} & c_{ss} & d_{sx} & d_{sy} & d_{ss}
\end{bmatrix}
$$

$$(4)$$

Subsequently, the entire laminate physical property calculation unit 21 of the control unit 20 obtains a total thickness (h) of the multilayer material, which is the entire laminate, from the thickness information of each layer (k), and calculates elastic moduli ($/E_{x,y}$), shear moduli ($/G_{x,y}$), and Poisson's ratios ($/\upsilon_{x,y}$) of the multilayer material, which is the entire laminate, using values of the compliance matrices ($[a]_{x,y}$, $[b]_{x,y}$, $[c]_{x,y}$, $[d]_{x,y}$) set above, as shown in Equation (5) below (S17).

$$
/E_x = \frac{1}{ha_{xx}} \qquad /E_y = \frac{1}{ha_{yy}} \qquad /G_{xy} = \frac{1}{ha_{ss}}
$$

$$
/V_{xy} = -\frac{a_{yx}}{a_{xx}} \qquad /V_{yx} = -\frac{a_{xy}}{a_{yy}}
$$

$$(5)$$

Next, the user inputs coefficients of thermal expansion ($\alpha^k_{1,2}$), coefficients of water expansion ($\beta^k_{1,2}$), a temperature change ($\Delta T$), and a humidity change ($\Delta C$) of each layer (k) using the input unit 10 while looking at an input screen (not shown) on the display 30 (S21), and then the expansion coefficient and warpage calculation unit 22 of the control unit 20 reads the input values.

Subsequently, the expansion coefficient and warpage calculation unit 22 of the control unit 20 calculates free lamina hydrothermal strains ($e^k_{1,2}$) caused by the water expansion of each layer (k) in the major direction of each layer (k) using the input coefficient of thermal expansion($\alpha^k_{1,2}$), coefficient of water expansion($\beta^k_{1,2}$), temperature change ($\Delta T$), and humidity change ($\Delta C$) of each layer (k) as shown in Equation (6) below (S22).

$$
e^k_1 = \alpha^k_1 \Delta T + \beta^k_1 \Delta C \qquad e^k_2 = \alpha^k_1 \Delta T + \beta^k_1 \Delta C
$$

$$(6)$$

Next, the expansion coefficient and warpage calculation unit 22 of the control unit 20 calculates hygrothermal strain transformations ($e^k_{x,y,s}$) of the multilayer material, which is the entire laminate, by reflecting a lamination angle of each layer (k) in the calculated free lamina hydrothermal strains as shown in Equation (7) below (S23).

$$e^k_x = e^k_1 m^2 + e^k_2 n^2 \qquad\qquad m = \cos\theta, \ n = \sin\theta$$

$$e^k_y = e^k_1 n^2 + e^k_2 m^2$$

$$e^k_s = 2(e^k_1 + e^k_2) mn \tag{7}$$

Subsequently, the expansion coefficient and warpage calculation unit 22 of the control unit 20 calculates hygrothermal forces ($N^{HT}_{x,y,s}$) and hygrothermal moments ($M^{HT}_{x,y,s}$), generated in the multilayer material, which is the entire laminate, based on the hygrothermal strain transformations ($e^k_{x,y,s}$) of the multilayer material, and the stiffness matrices ($[Q]^k_{x,y}$) of the multilayer material, which is the entire laminate, calculated in the second embodiment, and the thickness ($Z^k$) of each layer ($k$) as shown in Equation (8) below (S24).

$$
\begin{bmatrix} N^{HT}_x \\ N^{HT}_y \\ N^{HT}_s \end{bmatrix} = \sum_{k=1}^{n} \begin{bmatrix} Q_{xx} & Q_{xy} & Q_{xs} \\ Q_{xy} & Q_{yy} & Q_{ys} \\ Q_{xs} & Q_{ys} & Q_{ss} \end{bmatrix}_k \begin{bmatrix} e_x \\ e_y \\ e_s \end{bmatrix}_k t_k
$$

$$
\begin{bmatrix} M^{HT}_x \\ M^{HT}_y \\ M^{HT}_s \end{bmatrix} = \sum_{k=1}^{n} \begin{bmatrix} Q_{xx} & Q_{xy} & Q_{xs} \\ Q_{xy} & Q_{yy} & Q_{ys} \\ Q_{xs} & Q_{ys} & Q_{ss} \end{bmatrix}_k \begin{bmatrix} e_x \\ e_y \\ e_s \end{bmatrix}_k z_k t_k \tag{8}
$$

Then, the layer strain and stress calculation unit 22 of the control unit 20 forms total forces (/N) and total moments (/M) by adding external forces (N, M), which are mechanical loads, to the calculated hygrothermal forces ($N^{HT}_{x,y,s}$) and hygrothermal moments ($M^{HT}_{x,y,s}$) as shown in Equation (9) below (S25).

$$/N = N + N^{HT} \qquad /M = M + M^{HT} \tag{9}$$

Subsequently, the expansion coefficient and warpage calculation unit 22 of the control unit 20 obtains a coefficient of thermal expansion (/α) of the multilayer material, which is the entire laminate, using the total forces (/N) and the total moments (/M), and the compliance matrices ($[a]_{x,y}$, $[b]_{x,y}$, $[c]_{x,y}$, $[d]_{x,y}$) for the stiffness matrices ($[A]_{x,y}$, $[B]_{x,y}$, $[C]_{x,y}$) of the multilayer material, which is the entire laminate, calculated in the second embodiment as shown in Equation (10) below, and

$$
\begin{bmatrix} /\alpha_x \\ /\alpha_s \\ /\alpha_s \end{bmatrix} = \begin{bmatrix} \alpha_{xx} & \alpha_{xy} & \alpha_{xs} \\ \alpha_{yx} & \alpha_{yy} & \alpha_{ys} \\ \alpha_{sx} & \alpha_{sy} & \alpha_{ss} \end{bmatrix} \begin{bmatrix} N^T_x \\ N^T_y \\ N^T_s \end{bmatrix} + \begin{bmatrix} b_{xx} & b_{xy} & b_{xs} \\ b_{yx} & b_{yy} & b_{ys} \\ b_{sx} & b_{sy} & b_{ss} \end{bmatrix} \begin{bmatrix} M^T_x \\ M^T_y \\ M^T_s \end{bmatrix} \tag{10}
$$

(Here, provided that [N]=0, [M]=0, $\Delta T$=1)

also obtains a coefficient of water expansion (β) of the multilayer material, which is the entire laminate, as shown in Equation (11) below (S26).

$$
\begin{bmatrix} \overline{\beta}_x \\ \overline{\beta}_s \\ \overline{\beta}_s \end{bmatrix} = \begin{bmatrix} \alpha_{xx} & \alpha_{xy} & \alpha_{xs} \\ \alpha_{yx} & \alpha_{yy} & \alpha_{ys} \\ \alpha_{sx} & \alpha_{sy} & \alpha_{ss} \end{bmatrix} \begin{bmatrix} N_x^H \\ N_y^H \\ N_s^H \end{bmatrix} + \begin{bmatrix} b_{xx} & b_{xy} & b_{xs} \\ b_{yx} & b_{yy} & b_{ys} \\ b_{sx} & b_{sy} & b_{ss} \end{bmatrix} \begin{bmatrix} M_x^H \\ M_y^H \\ M_s^H \end{bmatrix}
$$

(11)

(Here, provided that [N]=0, [M]=0, ∆C=1)

Then, the expansion coefficient and warpage calculation unit 22 of the control unit 20 calculates strains ($\varepsilon^0_{x,y}$) and curvatures ($k_{x,y,s}$) of a middle plane using the total forces (/N) and the total moments (/M), and the compliance matrices ([a]$_{x,y}$, [b]$_{x,y}$, [c]$_{x,y}$, [d]$_{x,y}$) for the stiffness matrices ([A]$_{x,y}$, [B]$_{x,y}$, [C]$_{x,y}$) of the multilayer material, which is the entire laminate, calculated in the second embodiment as shown in Equation 12 below (S27).

$$
\begin{bmatrix} \epsilon_x^0 \\ \epsilon_y^0 \\ \gamma_s^0 \\ \hline K_x \\ K_y \\ K_s \end{bmatrix} = \begin{bmatrix} a_{xx} & a_{xy} & a_{xs} & b_{xx} & b_{xy} & b_{xs} \\ a_{yx} & a_{yy} & a_{ys} & b_{yx} & b_{yy} & b_{ys} \\ a_{sx} & a_{sy} & a_{ss} & b_{sx} & b_{sy} & b_{ss} \\ \hline c_{xx} & c_{xy} & c_{xs} & d_{xx} & d_{xy} & d_{xs} \\ c_{yx} & c_{yy} & c_{ys} & d_{yx} & d_{yy} & d_{ys} \\ c_{sx} & c_{sy} & c_{ss} & d_{sx} & d_{sy} & d_{ss} \end{bmatrix} \begin{bmatrix} /N_x \\ /N_y \\ /N_s \\ \hline /M_x \\ /M_y \\ /M_s \end{bmatrix}
$$

$$\overset{A \qquad\qquad B}{\underset{C \qquad\qquad D}{\phantom{X}}}$$

(12)

Subsequently, the expansion coefficient and warpage calculation unit 22 of the control unit 20 calculates a warpage (ω) by utilizing the curvatures ($k_{x,y,s}$) of the middle plate, and the sample size (x, y) information input through the input unit 10 as shown in Equation (13) below and then 3D plotted on an output image (S28).

$$
w = -\frac{1}{2}\left( k_x x^2 + k_y y^2 + k_s xy \right)
$$

(13)

On the output image provided in the display 30, as shown in FIG. 3, as stiffness homogenization results 31, a machine direction (MD) elastic modulus, a transverse (TD) elastic modulus, a Poisson's ratio, a shear modulus, a bulk modulus, and a thermal expansion rate are outputted, a warpage plot 32 is outputted, and a thickness-dependent x-direction strain 33, a thickness-dependent x-direction stress 34, a thickness-dependent y-direction strain 35, and a thickness-dependent y-direction stress 36 are output.

**(Fifth embodiment)**

In one embodiment of the present invention, when the values input to the input unit are not immediately obtained, they may be deduced through a conversion process using other physical property values.

In one embodiment, in the process of inputting the elastic modulus ($E^k$) of each layer (k) and the Poisson's ratio ($\upsilon^k$) of each layer (k), these values can be calculated using one or more physical property values of a Lame's first parameter ($\lambda^k$), a shear modulus ($G^k$) or a bulk elastic modulus ($K^k$). For example, they can be converted into an elastic modulus ($E^k$) and a Poisson's ratio ($\upsilon^k$) using Formulas 1 to 9 below.

When the available combination is ($\lambda^k$, $G^k$), it is expressed by Formula 1 below.

[Formula 1]

$$E^k = \frac{G^k(3\lambda^k + 2G^k)}{\lambda^k + G^k} \qquad \upsilon^k = \frac{\lambda^k}{2(\lambda^k + G^k)}$$

When the available combination is ($\lambda^k$, $E^k$), it is expressed by Formula 2 below.

[Formula 2]

$$\upsilon^k = \frac{A - (E^k + \lambda^k)}{4\lambda^k} \qquad E^k$$

When the available combination is ($\lambda^k$, $\upsilon^k$), it is expressed by Formula 3 below.

[Formula 3]

$$E^k = \frac{\lambda^k(1 + \lambda^k)(1 - 2\lambda^k)}{\lambda^k} \qquad \upsilon^k$$

When the available combination is ($\lambda^k$, $K^k$), it is expressed by Formula 4 below.

[Formula 4]

$$E^k = \frac{9K^k(K^k - \lambda^k)}{3K^k - \lambda^k} \qquad \upsilon^k = \frac{\lambda^k}{3K^k - \lambda^k}$$

When the available combination is ($G^k$, $E^k$), it is expressed by Formula 5 below.

[Formula 5]

$$\upsilon^k = \frac{E - 2G}{2G^k} \qquad E^k$$

When the available combination is ($G^k$, $\upsilon^k$), it is expressed by Formula 6 below.

[Formula 6]

$$E^k = 2G^k(1 + \upsilon^k) \qquad \upsilon^k$$

When the available combination is ($G^k$, $K^k$), it is expressed by Formula 7 below.

[Formula 7]

$$E^k = \frac{9K^k\,G^k}{3K^k + G^k} \qquad v^k = \frac{3K^k - 2G^k}{2(3K^k + G^k)}$$

When the available combination is ($K^k$, $E^k$), it is expressed by Formula 8 below.

[Formula 8]

$$v^k = \frac{3K^k - E^k}{6K^k} \qquad E^k$$

When the available combination is ($K^k$, $v^k$), it is expressed by Formula 9 below.

[Formula 9]

$$E^k = 3K^k(1 - 2v^k) \qquad v^k$$

The Formulas 1 to 9 above are examples, and it is possible to combine two or more formulas as needed.

[Description of reference numerals]

10 : Input unit
20 : Control unit
21 : Entire laminate physical property calculation unit
22 : Expansion coefficient and warpage calculation unit
30 : Display
40 : Storage unit

**Claims**

1. A system for predicting physical properties of a multilayer material having n laminated films (n is an integer of 2 or more), comprising:

an input unit configured for inputting input values including any one or more of an elastic modulus ($E^k$) of each layer (k), a Poisson's ratio ($v^k$) of each layer (k), a shear modulus ($G^k$) of each layer (k), a thickness ($Z^k$) of each layer (k), or a lamination angle ($\theta^k$) of each layer (k), coefficients of thermal expansion ($\alpha^k_{1,2}$) or coefficients of water expansion ($\beta^k_{1,2}$) of each layer (k), a temperature change ($\Delta T$), or a humidity change ($\Delta C$);
a control unit configured to calculate the physical properties of the multilayer material by applying input values to the input unit;
a display connected to the control unit; and
a storage unit connected to the control unit,
wherein the control unit is configured to calculate any one or more of a coefficient of thermal expansion ($\alpha$) of the multilayer material, a coefficient of water expansion ($\beta$) of the multilayer material, or a warpage of the multilayer material by processing values input to the input unit.

2. The system of claim 1, wherein the values input to the input unit comprise any one or more of elastic moduli ($E^k_{1,2}$) in the machine direction (1) or transverse direction (2) of each layer (k),

Poisson's ratios ($\upsilon^k_{1,2}$) in the machine direction (1) or the transverse direction (2) of each layer (k),
shear moduli ($G^K_{1,2}$) in the machine direction (1) or the transverse direction (2) of each layer (k),
an angle ($\theta^k$) in the machine direction (1) of each layer with respect to the x direction of the multilayer material, wherein the x direction means an arbitrarily set direction in a plane of the multilayer material,
a thickness ($Z^k$) of each layer (k); or
any one or more of coefficients of thermal expansion ($\alpha^k_{1,2}$), coefficients of water expansion ($\beta^k_{1,2}$), a temperature change ($\Delta T$), or a humidity change ($\Delta C$) of each layer (k).

3. The system of claim 1, wherein the input unit is configured for inputting elastic moduli ($E^k_{1,2}$) in a machine direction (1) and a transverse direction (2) of each layer (k), Poisson's ratios ($\upsilon^k_{1,2}$) in the machine direction (1) and the transverse direction (2) of each layer (k), shear moduli ($G^K_{1,2}$) in the machine direction (1) and the transverse direction (2) of each layer (k), an angle ($\theta^k$) in the machine direction (1) of each layer with respect to the x direction of the multilayer material, wherein the x direction means an arbitrarily set direction in a plane of the multilayer material, a thickness ($Z^k$) of each layer (k), coefficients of thermal expansion ($\alpha^k_{1,2}$), coefficients of water expansion ($\beta^k_{1,2}$), a temperature change ($\Delta T$), and a humidity change ($\Delta C$) of each layer (k).

4. The system of claim 1, wherein the control unit is configured to:

calculate stiffness matrices ($[Q]^k_{1,2}$) in the machine direction (1) and transverse direction (2) of each layer (k) using elastic moduli ($E^k_{1,2}$), Poisson's ratios ($\upsilon^k_{1,2}$), and shear moduli ($G^k_{1,2}$),
set inverse matrices ($[S]^k_{1,2}$) for the stiffness matrices ($[Q]^k_{1,2}$) in the machine direction (1) and the transverse direction (2) of each layer (k),
reset stiffness matrices ($[Q]^k_{x,y}$) of each layer (k) by reflecting a lamination angle ($\theta^k$) of the multilayer material in the stiffness matrices ($[Q]^k_{1,2}$),
calculate stiffness matrices ($[A]_{x,y}$, $[B]_{x,y}$, $[D]_{x,y}$) of the multilayer material using the values of the reset stiffness matrices by receiving the thickness information of each layer (k),
set compliance matrices ($[a]_{x,y}$, $[b]_{x,y}$, $[c]_{x,y}$, $[d]_{x,y}$) for the stiffness matrices ($[A]_{x,y}$, $[B]_{x,y}$, $[D]_{x,y}$) of the multilayer material,
calculate free lamina hydrothermal strains ($e^k_{1,2}$) generated by water expansion of each layer (k) in a major direction of each layer (k) using coefficients of thermal expansion ($\alpha^k_{1,2}$), coefficients of water expansion ($\beta^k_{1,2}$), a temperature change ($\Delta T$), and a humidity change ($\Delta C$) of each layer (k),
calculate hygrothermal strain transformations ($e^k_{x,y,s}$) of each layer (k) by reflecting a lamination angle ($\theta^k$) of the multilayer material in the free lamina hydrothermal strains ($e^k_{1,2}$),
calculate hygrothermal forces ($N^{HT}_{x,y,s}$) and hygrothermal moments ($M^{HT}_{x,y,s}$), generated in the multilayer material, based on the hygrothermal strain transformations ($e^k_{x,y,s}$) of the multilayer material, the stiffness matrices ($[Q]^k_{x,y}$) of the multilayer material, which is the entire laminate, and the thickness ($Z^k$) of each layer (k),
form total forces (/N) and total moments (/M) by adding external forces (N, M) to the hygrothermal forces ($N^{HT}_{x,y,s}$) and the hygrothermal moments ($M^{HT}_{x,y,s}$), and
calculates a coefficient of thermal expansion ($\alpha$) and coefficient of water expansion ($\beta$) of the multilayer material using the total forces (/N) and the total moments (/M), and the compliance matrices ($[a]_{x,y}$, $[b]_{x,y}$, $[c]_{x,y}$, $[d]_{x,y}$) for the stiffness matrices ($[A]_{x,y}$, $[B]_{x,y}$, $[D]_{x,y}$) of the multilayer material.

5. The system of claim 4, wherein the control unit is configured to calculate strains ($\varepsilon^0_{x,y}$) and curvatures ($k_{x,y,s}$) of a middle plane using the total forces (/N) and the total moments (/M), and the compliance matrices ($[a]_{x,y}$, $[b]_{x,y}$, $[c]_{x,y}$, $[d]_{x,y}$) for the stiffness matrices ($[A]_{x,y}$, $[B]_{x,y}$, $[D]_{x,y}$) of the multilayer material, and calculates a warpage of the multilayer material by utilizing the curvature ($k_{x,y,s}$) of the middle plane, and sample size (x, y) information.

6. The system of claim 1, wherein the control unit is further configured to calculate elastic moduli ($E_{x,y}$), shear moduli ($G_{x,y}$), and Poisson's ratios ($\upsilon_{x,y}$) of the multilayer material using the total thickness (h) of the multilayer material and the values of compliance matrices ($[a]_{x,y}$, $[b]_{x,y}$, $[c]_{x,y}$, $[d]_{x,y}$).

7. The system of claim 1, wherein the input unit is configured for inputting elastic moduli ($E^k_{1,2}$) in a machine direction (MD, 1) and a transverse direction (TD, 2) of each layer (k), Poisson's ratios ($\upsilon^k_{1,2}$) in the machine direction (1) and the transverse direction (2) of each layer (k), shear moduli ($G^K_{1,2}$) in the machine direction (1) and the transverse direction (2) of each layer (k), an angle ($\theta^k$) in the machine direction (1) of each layer with respect to the x direction of the multilayer material, wherein the x direction means an arbitrarily set direction in a plane of the multilayer material, a thickness ($Z^k$) of each layer (k), coefficients of thermal expansion ($\alpha^k_{1,2}$) and coefficients of water expansion ($\beta^k_{1,2}$) of each layer (k), a temperature change ($\Delta T$), and a humidity change ($\Delta C$).

8. The system of claim 1, wherein the control unit is configured to:

calculate stiffness matrices ($[Q]^k_{1,2}$) in the machine direction (1) and transverse direction (2) of each layer (k) using elastic moduli ($E^k_{1,2}$), Poisson's ratios ($\upsilon^k_{1,2}$), and shear moduli ($G^k_{1,2}$),
set inverse matrices ($[S]^k_{1,2}$) for the stiffness matrices ($[Q]^k_{1,2}$) in the machine direction (1) and the transverse direction (2) of each layer (k),
reset stiffness matrices ($[Q]^k_{x,y}$) of each layer (k) by reflecting a lamination angle ($\theta^k$) of the multilayer material in the stiffness matrices ($[Q]^k_{1,2}$),
calculate stiffness matrices ($[A]_{x,y}$, $[B]_{x,y}$, $[D]_{x,y}$) of the multilayer material using the values of the reset stiffness matrices by receiving the thickness information of each layer (k),
set compliance matrices ($[a]_{x,y}$, $[b]_{x,y}$, $[c]_{x,y}$, $[d]_{x,y}$) for the stiffness matrices ($[A]_{x,y}$, $[B]_{x,y}$, $[D]_{x,y}$) of the multilayer material,
calculate elastic moduli ($E_{x,y}$), shear moduli ($G_{x,y}$), and Poisson's ratios ($\upsilon_{x,y}$) of the multilayer material using the total thickness (h) of the multilayer material and the values of the compliance matrices ($[a]_{x,y}$, $[b]_{x,y}$, $[c]_{x,y}$, $[d]_{x,y}$),
calculate free lamina hydrothermal strains ($e^k_{1,2}$) generated by water expansion of each layer (k) in a major direction of each layer (k) using coefficients of thermal expansion ($\alpha^k_{1,2}$), coefficients of water expansion ($\beta^k_{1,2}$), a temperature change ($\Delta T$), and a humidity change ($\Delta C$) of each layer (k),
calculate hygrothermal strain transformations ($e^k_{x,y,s}$) of each layer (k) by reflecting a lamination angle ($\theta^k$) of each layer (k) in the free lamina hydrothermal strains,
calculate hygrothermal forces ($N^{HT}_{x,y,s}$) and hygrothermal moments ($M^{HT}_{x,y,s}$) generated in the multilayer material based on the hygrothermal strain transformations ($e^k_{x,y,s}$) of each layer (k), the stiffness matrices ($[Q]^k_{x,y}$) of each layer (k), and the thickness ($Z^k$) of each layer (k),
form total forces (/N) and total moments (/M) by adding external forces (N, M) to the hygrothermal forces ($N^{HT}_{x,y,s}$) and the hygrothermal moments ($M^{HT}_{x,y,s}$),
calculate a coefficient of thermal expansion ($\alpha$) and coefficient of water expansion ($\beta$) of the multilayer material using the total forces (/N) and the total moments (/M), and the compliance matrices ($[a]_{x,y}$, $[b]_{x,y}$, $[c]_{x,y}$, $[d]_{x,y}$) for the stiffness matrices ($[A]_{x,y}$, $[B]_{x,y}$, $[D]_{x,y}$) of the multilayer material,
calculate strains ($\varepsilon^0_{x,y}$) and curvatures ($k_{x,y,s}$) of a middle plane using the total forces (/N) and the total moments (/M), and the compliance matrices ($[a]_{x,y}$, $[b]_{x,y}$, $[c]_{x,y}$, $[d]_{x,y}$) for the stiffness matrices ($[A]_{x,y}$, $[B]_{x,y}$, $[D]_{x,y}$) of the multilayer material, and
calculate a warpage of the multilayer material by utilizing the curvature ($k_{x,y,s}$) of the middle plane, and sample size (x, y) information.

9. A method of predicting the physical properties of a multilayer material having n laminated films (n is an integer of 2 or more), comprising:

inputting input values including any one or more of an elastic modulus ($E^k$) of each layer (k), a Poisson's ratio ($\upsilon^k$) of each layer (k), a shear modulus ($G^k$) of each layer (k), a thickness ($Z^k$) of each layer (k), or a lamination angle ($\theta^k$) of each layer (k), coefficients of thermal expansion ($\alpha^k_{1,2}$) or coefficients of water expansion ($\beta^k_{1,2}$) of each layer (k), a temperature change ($\Delta T$), or a humidity change ($\Delta C$); and
calculating any one or more output values of a coefficient of thermal expansion ($\alpha$) of the multilayer material, a coefficient of water expansion ($\beta$) of the multilayer material, or a warpage of the multilayer material by applying the input values.

10. The method of claim 9, wherein, in the inputting of input values,

the input values comprise any one or more of elastic moduli ($E^k_{1,2}$) in the machine direction (1) or transverse direction (2) of each layer (k),
Poisson's ratios ($\upsilon^k_{1,2}$) in the machine direction (1) or transverse direction (2) of each layer (k),
shear moduli ($G^K_{1,2}$) in the machine direction (1) or transverse direction (2) of each layer (k),
an angle ($\theta^k$) in the machine direction (1) of each layer with respect to the x direction of the multilayer material, wherein the x direction means an arbitrarily set direction in a plane of the multilayer material, and
a thickness ($Z^k$) of each layer (k); and

any one or more of coefficients of thermal expansion ($\alpha^k_{1,2}$), coefficients of water expansion ($\beta^k_{1,2}$), a temperature change ($\Delta T$), or a humidity change ($\Delta C$) of each layer (k).

11. The method of claim 9, wherein the inputting of input values comprises inputting elastic moduli ($E^k_{1,2}$) in the machine direction (1) and transverse direction (2) of each layer (k), Poisson's ratios ($\upsilon^k_{1,2}$) in the machine direction (1) and the transverse direction (2) of each layer (k), shear moduli ($G^K_{1,2}$) in the machine direction (1) and the transverse direction (2) of each layer (k), an angle ($\theta^k$) in the machine direction (1) of each layer (k) with respect to the x direction of the multilayer material, and a thickness ($Z^k$) of each layer (k).

12. The method of claim 9, wherein the calculating of output values comprises calculating stiffness matrices ($[Q]^k_{1,2}$) in the machine direction (1) and transverse direction (2) of each layer (k) using elastic moduli ($E^k_{1,2}$), Poisson's ratios ($\upsilon^k_{1,2}$), and shear moduli ($G^k_{1,2}$);

   setting inverse matrices ($[S]^k_{1,2}$) for the stiffness matrices ($[Q]^k_{1,2}$) in the machine direction (1) and the transverse direction (2) of each layer (k);
   resetting stiffness matrices ($[Q]^k_{x,y}$) of the multilayer material by reflecting a lamination angle ($\theta^k$) of each layer (k) in the stiffness matrices ($[Q]^k_{1,2}$);
   calculating stiffness matrices ($[A]_{x,y}$, $[B]_{x,y}$, $[D]_{x,y}$) of the multilayer material using the values of the reset stiffness matrices by receiving the thickness information of each layer (k);
   setting compliance matrices ($[a]_{x,y}$, $[b]_{x,y}$, $[c]_{x,y}$, $[d]_{x,y}$) for the stiffness matrices ($[A]_{x,y}$, $[B]_{x,y}$, $[D]_{x,y}$) of the multilayer material;
   inputting coefficients of thermal expansion ($\alpha^k_{1,2}$), coefficients of water expansion ($\beta^k_{1,2}$), a temperature change ($\Delta T$), and a humidity change ($\Delta C$) of each layer (k);
   calculating free lamina hydrothermal strains ($e^k_{1,2}$) generated by water expansion of each layer (k) in a major direction of each layer (k) using the coefficients of thermal expansion ($\alpha^k_{1,2}$), coefficients of water expansion ($\beta^k_{1,2}$), temperature change ($\Delta T$), and humidity change ($\Delta C$) of each layer (k);
   calculating hygrothermal strain transformations ($e^k_{x,y,s}$) of the multilayer material by reflecting a lamination angle ($\theta^k$) of the multilayer material in the free lamina hydrothermal strains ($e^k_{1,2}$);
   calculating hygrothermal forces ($N^{HT}_{x,y,s}$) and hygrothermal moments ($M^{HT}_{x,y,s}$), generated in the multilayer material, based on the hygrothermal strain transformations ($e^k_{x,y,s}$) of the multilayer material, the stiffness matrices ($[Q]^k_{x,y}$) of the multilayer material, which is the entire laminate, and the thickness ($Z^k$) of each layer (k);
   forming total forces (/N) and total moments (/M) by adding external forces (N, M) to the hygrothermal forces ($N^{HT}_{x,y,s}$) and the hygrothermal moments ($M^{HT}_{x,y,s}$); and
   calculating a coefficient of thermal expansion ($\alpha$) and coefficient of water expansion ($\beta$) of the multilayer material using the total forces (/N) and the total moments (/M), and the compliance matrices ($[a]_{x,y}$, $[b]_{x,y}$, $[c]_{x,y}$, $[d]_{x,y}$) for the stiffness matrices ($[A]_{x,y}$, $[B]_{x,y}$, $[D]_{x,y}$) of the multilayer material.

13. The method of claim 12, further comprising:

   calculating calculates strains ($\varepsilon^0_{x,y}$) and curvatures ($k_{x,y,s}$) of a middle plane using the total forces (/N) and the total moments (/M), and the compliance matrices ($[a]_{x,y}$, $[b]_{x,y}$, $[c]_{x,y}$, $[d]_{x,y}$) for the stiffness matrices ($[A]_{x,y}$, $[B]_{x,y}$, $[D]_{x,y}$) of the multilayer material; and
   calculating a warpage of the multilayer material by utilizing the curvature ($k_{x,y,s}$) of the middle plane, and sample size (x, y) information.

14. The method of claim 12, further comprising:

   after the setting of the compliance matrices ($[a]_{x,y}$, $[b]_{x,y}$, $[c]_{x,y}$, $[d]_{x,y}$) (S16),
   calculating elastic moduli ($E_{x,y}$), shear moduli ($G_{x,y}$), and Poisson's ratios ($\upsilon_{x,y}$) of the multilayer material using the total thickness (h) of the multilayer material and the values of the compliance matrices ($[a]_{x,y}$, $[b]_{x,y}$, $[c]_{x,y}$, $[d]_{x,y}$).

15. The method of claim 9, wherein the inputting of input values comprises inputting elastic moduli ($E^k_{1,2}$) in the machine direction (1) and transverse direction (2) of each layer (k), Poisson's ratios ($\upsilon^k_{1,2}$) in the machine direction (1) and the transverse direction (2) of each layer (k), shear moduli ($G^K_{1,2}$) in the machine direction (1) and the transverse direction (2) of each layer (k), an angle ($\theta^k$) in the machine direction (1) of each layer (k) with respect to the x direction of the multilayer material, and a thickness ($Z^k$) of each layer (k).

16. The method of claim 9, wherein the calculating of output values comprises calculating stiffness matrices ($[Q]^k_{1,2}$)

in the machine direction (1) and transverse direction (2) of each layer (k) using elastic moduli ($E^k_{1,2}$), Poisson's ratios ($\upsilon^k_{1,2}$), and shear moduli ($G^k_{1,2}$);

setting inverse matrices ($[S]^k_{1,2}$) for the stiffness matrices ($[Q]^k_{1,2}$) in the machine direction (1) and the transverse direction (2) of each layer (k);

resetting stiffness matrices ($[Q]^k_{x,y}$) of the multilayer material by reflecting a lamination angle ($\theta^k$) of each layer (k) in the stiffness matrices ($[Q]^k_{1,2}$);

calculating stiffness matrices ($[A]_{x,y}$, $[B]_{x,y}$, $[D]_{x,y}$) of the multilayer material using the values of the reset stiffness matrix by receiving the thickness information of each layer (k);

setting compliance matrices ($[a]_{x,y}$, $[b]_{x,y}$, $[c]_{x,y}$, $[d]_{x,y}$) for the stiffness matrices ($[A]_{x,y}$, $[B]_{x,y}$, $[D]_{x,y}$) of the multilayer material;

calculating elastic moduli ($E_{x,y}$), shear moduli ($G_{x,y}$), and Poisson's ratios ($\upsilon_{x,y}$) of the multilayer material using the total thickness (h) of the multilayer material and the values of the compliance matrices ($[a]_{x,y}$, $[b]_{x,y}$, $[c]_{x,y}$, $[d]_{x,y}$);

inputting coefficients of thermal expansion ($\alpha^k_{1,2}$), coefficients of water expansion ($\beta^k_{1,2}$), a temperature change ($\Delta T$), and a humidity change ($\Delta C$) of each layer (k);

calculating free lamina hydrothermal strains ($e^k_{1,2}$) generated by water expansion of each layer (k) in a major direction of each layer (k) using the coefficients of thermal expansion ($\alpha^k_{1,2}$), coefficients of water expansion ($\beta^k_{1,2}$), temperature change ($\Delta T$), and humidity change ($\Delta C$) of each layer (k);

calculating hygrothermal strain transformations ($e^k_{x,y,s}$) of the multilayer material by reflecting a lamination angle ($\theta^k$) of the multilayer material in the free lamina hydrothermal strains ($e^k_{1,2}$);

calculating hygrothermal forces ($N^{HT}_{x,y,s}$) and hygrothermal moments ($M^{HT}_{x,y,s}$), generated in the multilayer material, based on the hygrothermal strain transformations ($e^k_{x,y,s}$) of the multilayer material, the stiffness matrices ($[Q]^k_{x,y}$) of the multilayer material, which is the entire laminate, and the thickness ($Z^k$) of each layer (k);

forming total forces (/N) and total moments (/M) by adding external forces (N, M) to the hygrothermal forces ($N^{HT}_{x,y,s}$) and the hygrothermal moments ($M^{HT}_{x,y,s}$);

calculating a coefficient of thermal expansion ($\alpha$) and coefficient of water expansion ($\beta$) of the multilayer material using the total forces (/N) and the total moments (/M), and the compliance matrices ($[a]_{x,y}$, $[b]_{x,y}$, $[c]_{x,y}$, $[d]_{x,y}$) for the stiffness matrices ($[A]_{x,y}$, $[B]_{x,y}$, $[D]_{x,y}$) of the multilayer material;

calculating calculates strains ($\varepsilon^0_{x,y}$) and curvatures ($k_{x,y,s}$) of a middle plane using the total forces (/N) and the total moments (/M), and the compliance matrices ($[a]_{x,y}$, $[b]_{x,y}$, $[c]_{x,y}$, $[d]_{x,y}$) for the stiffness matrices ($[A]_{x,y}$, $[B]_{x,y}$, $[D]_{x,y}$) of the multilayer material; and

calculating a warpage of the multilayer material by utilizing the curvature ($k_{x,y,s}$) of the middle plane, and sample size (x, y) information.

【FIG. 1】

**[FIG. 2]**

EP 4 209 773 A1

### Input Information (11)

| LAMINATION INFORMATION | 1ST LAYER | 2ND LAYER | 3RD LAYER |
|---|---|---|---|
| Name | | | |
| Thickness (um) | 50 | 100 | 60 |
| Ply-Angle | 0 | 0 | 0 |

STIFFNESS PREDICTION INPUT (12)

| | 1ST LAYER | 2ND LAYER | 3RD LAYER |
|---|---|---|---|
| Elastic Modules (Mpa, MD) | 200 | 400 | 70 |
| Elastic Modules (Mpa, TD) | 200 | 400 | 78 |
| Poisson's Ratio | 0.3 | 0.3 | 0.25 |
| Thermal Expansion(option) | 10.4 | 8.5 | 0 |

STIFFNESS HOMOGENIZATION

113 112 111
121 122 123 124

### Parameters (13)

SAMPLE SIZE

100mm × 100mm (131)

EXTERNAL INPUT (14)

| | |
|---|---|
| X-AXIS STRETCHING (Mpa) | 10 (141) |
| Y-AXIS STRETCHING (Mpa) | 0 (142) |
| Shear (Mpa) | 0 (143) |
| TEMPERATURE CHANGE | 20 (144) |

【FIG. 3】

【FIG. 4】

$$E^k_{1.2}, \upsilon^k_{1.2}, G^k_{1.2}, \theta^k_{1.2}, Z^k \text{ INPUT} \quad \sim S11$$

$S13 \sim \quad [S]^k_{1.2} \quad \longleftrightarrow \quad [Q]^k_{1.2} \quad \sim S12$

$$\theta_k$$

$$[Q]^k_{x,y} \quad \sim S14$$

$$Z_k$$

$$[A]_{x,y}, [B]_{x,y}, [D]_{x,y} \quad \sim S15$$

$$[a]_{x,y} \quad , \quad [b]_{x,y}$$
$$[c]_{x,y} \quad , \quad [d]_{x,y} \quad \sim S16$$

$$h$$

$$/E_{x,y} \, , \, /V_{x,y} \, , \, /G_{x,y} \quad \sim S17$$

【FIG. 5】

$$\alpha^k_{1,2}, \beta^k_{1,2}, \Delta T, \Delta C \ \text{INPUT} \quad \text{—S21}$$

$$e^k_{1,2} \quad \text{—S22}$$

$$\theta_k$$

$$e^k_{x,y,s} \quad \text{—S23}$$

$$[Q]^k_{x,y}$$

$$Z_k$$

$$N^{HT}, M^{HT} \quad \text{—S24}$$

$$N, M$$

$$/N, /M \quad \text{—S25}$$

S26— COEFFICIENTS OF THERMAL EXPANSION AND WATER EXPANSION OF LAMINATE

$$[a]_{x,y}, [b]_{x,y}$$
$$[c]_{x,y}, [d]_{x,y}$$

CALCULATION OF STRAINS AND CURVATURES OF MIDDLE PLANE —S27

SAMPLE SIZE

WARPAGE 3D PLOTTING —S28

## INTERNATIONAL SEARCH REPORT

| International application No. |
|---|
| **PCT/KR2022/012499** |

**A. CLASSIFICATION OF SUBJECT MATTER**

**G01N 3/00**(2006.01)i; **G01N 3/06**(2006.01)i; **G01N 3/08**(2006.01)i; **G01N 3/24**(2006.01)i; **G01N 3/60**(2006.01)i; **G01N 33/44**(2006.01)i

According to International Patent Classification (IPC) or to both national classification and IPC

**B. FIELDS SEARCHED**

Minimum documentation searched (classification system followed by classification symbols)

G01N 3/00(2006.01); B29C 70/02(2006.01); B32B 37/00(2006.01); B32B 5/00(2006.01); E04C 3/02(2006.01); G01N 3/42(2006.01); G06F 17/10(2006.01); G06F 17/50(2006.01); G06F 19/00(2011.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 다층 소재(multilayer material), 물성(physical property), 예측(predict), 탄성 계수 (modulus of elasticity), 포아송비(Poisson`s ratio), 전단 계수(shear modulus), 열팽창 계수(coefficient of thermal expansion), 두께(thickness), 강성 매트릭스(stiffness matrix), 역행렬(compliance matrix)

**C. DOCUMENTS CONSIDERED TO BE RELEVANT**

| Category* | Citation of document, with indication, where appropriate, of the relevant passages | Relevant to claim No. |
|---|---|---|
| Y | KR 10-1780173 B1 (SABIC GLOBAL TECHNOLOGIES B.V.) 19 September 2017 (2017-09-19) See paragraphs [0025]-[0111], claims 1, 11 and 17 and figures 1-8. | 1-3,7,9-11,15 |
| A | | 4-6,8,12-14,16 |
| Y | JP 6625306 B1 (MITSUBISHI ELECTRIC CORPORATION) 25 December 2019 (2019-12-25) See paragraphs [0014], [0030] and [0050]-[0080] and figures 1-11. | 1-3,7,9-11,15 |
| A | KR 10-2016-0119393 A (INDUSTRY-ACADEMIC COOPERATION FOUNDATION, YONSEI UNIVERSITY) 13 October 2016 (2016-10-13) See claims 1-7 and figure 1. | 1-16 |
| A | KR 10-2005-0112788 A (DAEYANG FOUNDATION et al.) 01 December 2005 (2005-12-01) See paragraphs [0020]-[0143], claims 1-4 and figures 2a-7. | 1-16 |

☑ Further documents are listed in the continuation of Box C.    ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **13 December 2022** | **13 December 2022** |

| Name and mailing address of the ISA/KR | Authorized officer |
|---|---|
| **Korean Intellectual Property Office** Government Complex-Daejeon Building 4, 189 Cheongsa-ro, Seo-gu, Daejeon 35208 | |
| Facsimile No. **+82-42-481-8578** | Telephone No. |

Form PCT/ISA/210 (second sheet) (July 2022)

**INTERNATIONAL SEARCH REPORT**

International application No.

**PCT/KR2022/012499**

| C. | DOCUMENTS CONSIDERED TO BE RELEVANT | | |
| --- | --- | --- |
| Category* | Citation of document, with indication, where appropriate, of the relevant passages | Relevant to claim No. |
| A | KR 10-1200167 B1 (KOREA AEROSPACE INDUSTRIES, LTD.) 13 November 2012 (2012-11-13)<br>See paragraphs [0036]-[0082] and figures 1 and 2. | 1-16 |

**INTERNATIONAL SEARCH REPORT**
Information on patent family members

International application No.

**PCT/KR2022/012499**

| Patent document cited in search report | | | Publication date (day/month/year) | Patent family member(s) | | | Publication date (day/month/year) |
|---|---|---|---|---|---|---|---|
| KR | 10-1780173 | B1 | 19 September 2017 | CN | 106796617 | A | 31 May 2017 |
| | | | | CN | 106796617 | B | 25 September 2018 |
| | | | | EP | 3180193 | A1 | 21 June 2017 |
| | | | | JP | 2018-156689 | A | 04 October 2018 |
| | | | | JP | 2018-503884 | A | 08 February 2018 |
| | | | | JP | 6356339 | B2 | 11 July 2018 |
| | | | | US | 2017-0371980 | A1 | 28 December 2017 |
| | | | | WO | 2017-027598 | A1 | 16 February 2017 |
| JP | 6625306 | B1 | 25 December 2019 | EP | 3960452 | A1 | 02 March 2022 |
| | | | | EP | 3960452 | A4 | 27 April 2022 |
| | | | | JP | 2021-217281 | A1 | 06 May 2021 |
| | | | | US | 2022-0156439 | A1 | 19 May 2022 |
| | | | | WO | 2020-217281 | A1 | 29 October 2020 |
| KR | 10-2016-0119393 | A | 13 October 2016 | KR | 10-1677840 | B1 | 21 November 2016 |
| KR | 10-2005-0112788 | A | 01 December 2005 | KR | 10-0612032 | B1 | 11 August 2006 |
| KR | 10-1200167 | B1 | 13 November 2012 | KR | 10-1102307 | B1 | 05 January 2012 |
| | | | | KR | 10-2010-0119055 | A | 09 November 2010 |
| | | | | KR | 10-2012-0082112 | A | 23 July 2012 |

31

**REFERENCES CITED IN THE DESCRIPTION**

**Patent documents cited in the description**

- KR 1020210113915
- KR 1020210138918
- KR 1020220086165